# EUROPEAN PATENT SPECIFICATION

(11) **EP 0 959 894 B1**
(45) Date of publication and mention of the grant of the patent: **13.10.2004**
(21) Application number: 97928863.6
(22) Date of filing: 10.06.1997
(51) Int. Cl.: A61K 38/00, A61K 38/04, C07H 21/02, C07K 16/00, C12N 1/20, C12N 5/00, C12N 15/00

(54) **USE OF SUBSTRATE SUBTRACTION LIBRARIES TO DISTINGUISH ENZYME SPECIFICITIES**
VERWENDUNG VON SUBSTRAT-SUBTRAKTIONSBIBLIOTHEKEN ZUR UNTERSCHEIDUNG VON ENZYMSPEZIFITÄTEN
UTILISATION DE BANQUES DE SOUSTRACTION DE SUBSTRATS POUR DISTINGUER LES SPECIFICITES D'ENZYMES

(30) Priority: 10.06.1996 US 19495 P
(43) Date of publication of application: 01.12.1999
(73) Proprietor: The Scripps Research Institute, La Jolla, CA 92037 (US)
(72) Inventor: MADISON, Edwin, L., Del Mar, CA 92014 (US); KE, Song-Hua, San Diego, CA 92122 (US)
(74) Representative: Fisher, Adrian John
(86) International application number: PCT/US1997/009760
(87) International publication number: WO 1997/047314

(56) References cited:
- US-A- 5 492 894
- LI DING ET AL.: "Origins of the specificity of tissue-type plasminogen activator" PROCEEDINGS OF THE NATIONAL ACADEMY OF SCIENCES OF USA., vol. 92, no. 17, 15 August 1995 (1995-08-15), pages 7627-7631, XP002141767 NATIONAL ACADEMY OF SCIENCE. WASHINGTON., US ISSN: 0027-8424
- SONG-HUA KE ET AL.: "Distinguishing the specificities of closely related proteases" JOURNAL OF BIOLOGICAL CHEMISTRY., vol. 272, no. 26, 27 June 1997 (1997-06-27), pages 16603-16609, XP002141768 AMERICAN SOCIETY OF BIOLOGICAL CHEMISTS, BALTIMORE, MD., US ISSN: 0021-9258
- ANTI-CANCER DRUG DESIGN, January 1997, Vol. 12, LAM K.S., "Application of Combinatorial Library Methods in Cancer Research and Drug Discovery", pages 145-167.
- NATURE, 07 November 1991, Vol. 354, LAM et al., "A New Type of Synthetic Peptide Library for Identifying Ligand-Binding Activity", pages 82-84.
- NATURE, 07 November 1991, Vol. 354, HOUGHTEN et al., "Generation and Use of Synthetic Peptide Combinatorial Libraries for Basic Research and Drug Discovery", pages 84-86.
- NATURE, 17 March 1994, Vol. 368, BRONNER et al., "Mutation in the DNA Mismatch Repair Gene Homologue hMLH1 is Associated with Hereditary Non-Polyposis Colon Cancer", pages 258-261.
- GENE, 1989, Vol. 85, BATES et al., "Characterization of a Cryptic Plasmid from Lactobacillus Plantarum", pages 253-258.
- J. BIOL. CHEM., 25 April 1991, Vol. 266, No. 12, RAMSEIER et al., "Discovery and Sequence Analysis of Bacterial Genes Involved in the Biogenesis of c-Type Cytochromes", pages 7793-7803.
- MOLECULAR AND GENERAL GENETICS, June 1989, Vol. 217, No. 2/3, HIRATSUKA et al., "The Complete Sequence of the Rice (Oryza Sativa) Chloroplast Genome: Intermolecular Recombination Between Distinct tRNA Genes Accounts for a Major Plastid DNA Inversion During the Evolution of the Cereals", pages 185-194.
- J. BIOL. CHEM., 15 September 1995, Vol. 270, No. 37, DUNCAN et al., "Alternative Splicing of STY, a Nuclear Dual Specificity Kinase", pages 21524-21531.
- NUCLEIC ACIDS RESEARCH, December 1995, Vol. 23, No. 24, LACOSTE et al., "Characterization and Cloning of p11, a Transrepressor of Drosophila Melanogaster Retrotransposon 1731", pages 5073-5079.
- JOURNAL OF MOLECULAR BIOLOGY, 05 December 1990, Vol. 216, No. 3, KURAMOCHI et al., "Characterization of Murine Erythropoietin Receptor Genes", pages 567-575.
- J. BIOL. CHEM., 05 January 1993, Vol. 268, No. 1, AKIYAMA et al., "An Exopolyphosphatase of Escherichia Coli: The Enzyme and It's ppx Gene in a Polyphosphate Operon", pages 633-639.
- JOURNAL OF BACTERIOLOGY, February 1994, Vol. 176, No. 3, DE GROOT et al., "Characterization of Type IV Pilus Genes in Plant Growth-Promoting Pseudomonas Putida WCS358", pages 642-650.

### SEQUENCE LISTING

(1) GENERAL INFORMATION:
   (i) APPLICANT: Madison, Edwin L.
      Ke, Song-hua
   (ii) TITLE OF INVENTION: USE OF SUBSTRATE SUBTRACTION LIBRARIES TO DISTINGUISH ENZYME SPECIFICITIES
   (iii) NUMBER OF SEQUENCES:
   (iv) CORRESPONDENCE ADDRESS:
      (A) ADDRESSEE: Olson & Hierl, Ltd.
      (B) STREET: 20 North Wacker Drive, 36th Floor
      (C) CITY: Chicago
      (D) STATE: IL
      (E) COUNTRY: US
      (F) ZIP: 60606
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: WordPerfect 5.1 (ASCII File)
   (vi) CURRENT APPLICATION DATA:
      (A) APPLICATION NUMBER:
      (B) FILING DATE: 10-JUN-1997
      (C) CLASSIFICATION:
   (vii) PRIOR APPLICATION DATA:
      (A) APPLICATION NUMBER: US 60/019,495
      (B) FILING DATE: 10-JUN-1996
   (viii) ATTORNEY/AGENT INFORMATION:
      (A) NAME: Olson, Arne M
      (B) REGISTRATION NUMBER: 30,203
      (C) REFERENCE/DOCKET NUMBER: TSRI543.1PCT
   (ix) TELECOMMUNICATION INFORMATION:
      (A) TELEPHONE: 312-580-1180
      (B) TELEFAX: 312-580-1189
(2) INFORMATION FOR SEQ ID NO:1:
   (i) SEQUENCE CHARACTERISTICS:
      (A) LENGTH: 14 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:1:
   Lys Lys Ser Pro Gly Arg Val Val Gly Gly Ser Val Ala His          14
   1               5                   10
(2) INFORMATION FOR SEQ ID NO:2:
   (i) SEQUENCE CHARACTERISTICS:
      (A) LENGTH: 13 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:2:
   Leu Gly Gly Ser Gly Gln Arg Gly Arg Lys Ala Leu Glu 13
   1 5 10
(2) INFORMATION FOR SEQ ID NO:3:
   (i) SEQUENCE CHARACTERISTICS:
      (A) LENGTH: 13 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:3:
   Leu Gly Gly Ser Gly Glu Arg Ala Arg Gly Ala Leu Glu 13
   1 5 10
(2) INFORMATION FOR SEQ ID NO:4:
   (i) SEQUENCE CHARACTERISTICS:
      (A) LENGTH: 13 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:4:
   Leu Gly Gly Ser Gly His Tyr Gly Arg Ser Gly Leu Glu 13
   1 5 10
(2) INFORMATION FOR SEQ ID NO:5:
   (i) SEQUENCE CHARACTERISTICS:
      (A) LENGTH: 4 amino acids
      (B) TYPE: amino acid
      (C) STRANDEDNESS:not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:5:
   Tyr Gly Arg Ser                                                    4
   1
(2) INFORMATION FOR SEQ ID NO:6:
   (i) SEQUENCE CHARACTERISTICS:
      (A) LENGTH: 4 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:6:
   Arg Gly Arg Lys                                                    4
   1
(2) INFORMATION FOR SEQ ID NO:7:
   (i) SEQUENCE CHARACTERISTICS:
      (A) LENGTH: 5 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:7:
   Phe Arg Gly Arg Lys                                         5
   1               5
(2) INFORMATION FOR SEQ ID NO:8:
   (i) SEQUENCE CHARACTERISTICS:
      (A) LENGTH: 13 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:8:
   Leu Gly Gly Tyr Gly Arg Ser Ala Asn Ala Ile Leu Glu         13
   1               5                   10
(2) INFORMATION FOR SEQ ID NO:9:
   (i) SEQUENCE CHARACTERISTICS:
      (A) LENGTH: 13 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:9:
   Leu Gly Gly Arg Gly Arg Ser Ala Asn Ala Ile Leu Glu 13
   1 5 10
(2) INFORMATION FOR SEQ ID NO:10:
   (i) SEQUENCE CHARACTERISTICS:
      (A) LENGTH: 13 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:10:
   Leu Gly Gln Arg Gly Arg Ser Ala Asn Ala Ile Leu Glu 13
   1 5 10
(2) INFORMATION FOR SEQ ID NO:11:
   (i) SEQUENCE CHARACTERISTICS:
      (A) LENGTH: 13 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:11:
   Leu Gly Gly Ser Gly Arg Ser Ala Asn Ala Ile Leu Glu 13
   1 5 10
(2) INFORMATION FOR SEQ ID NO: 12:
   (i) SEQUENCE CHARACTERISTICS:
      (A) LENGTH: 13 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:12:
   Leu Gly Gly Ser Gly Arg Asn Ala Gln Val Arg Leu Glu          13
   1               5                   10
(2) INFORMATION FOR SEQ ID NO:13:
   (i) SEQUENCE CHARACTERISTICS:
      (A) LENGTH: 13 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:13:
   Leu Gly Gly Ser Gly Arg Ser Ala Thr Arg Asp Leu Glu          13
   1               5                   10
(2) INFORMATION FOR SEQ ID NO:14:
   (i) SEQUENCE CHARACTERISTICS:
      (A) LENGTH: 13 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:14:
   Leu Gly Gly Ser Gly Arg Lys Ala Ser Leu Ser Leu Glu        13
   1               5                   10
(2) INFORMATION FOR SEQ ID NO:15:
   (i) SEQUENCE CHARACTERISTICS:
      (A) LENGTH: 4 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:15:
   Ser Gly Arg Ser        4
   1
(2) INFORMATION FOR SEQ ID NO:16:
   (i) SEQUENCE CHARACTERISTICS:
      (A) LENGTH: 5 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:16:
   Ser Gly Arg Ser Ala 5
   1 5
(2) INFORMATION FOR SEQ ID NO:17:
   (i) SEQUENCE CHARACTERISTICS:
      (A) LENGTH: 4 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:17:
   Ser Gly Lys Ser 4
   1
(2) INFORMATION FOR SEQ ID NO:18
   (i) SEQUENCE CHARACTERISTICS:
      (A) LENGTH: 5 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:18:
   Gly Ser Gly Lys Ser 5
   1 5
(2) INFORMATION FOR SEQ ID NO:19:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:19:
   Ala Leu Arg Arg Gly Asp                                          6
   1               5
(2) INFORMATION FOR SEQ ID NO:20:
   (i) SEQUENCE CHARACTERISTICS:
      (A) LENGTH: 7 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:20:
   Asp Tyr Arg Gly Arg Met Leu                                      7
   1               5
(2) INFORMATION FOR SEQ ID NO:21:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:21:
   Glu Arg Ala Arg Gly Ala                                                    6
   1               5
(2) INFORMATION FOR SEQ ID NO:22:
   (i) SEQUENCE CHARACTERISTICS:
      (A) LENGTH: 6 amino acids
      (B) TYPE: amino acid
      (C) STRANDEDNESS: not: relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:22:
   Glu Arg Leu Arg Lys Ala                                                    6
   1               5
(2) INFORMATION FOR SEQ ID NO:23:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO: 23:
   Phe Gly Arg His Ala Ala 6
   1 5
(2) INFORMATION FOR SEQ ID NO:24:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:24:
   Phe Leu Pro Arg Thr Ala 6
   1 5
(2) INFORMATION FOR SEQ ID NO:25:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:25:
   Phe Arg Gly Arg Ala Ala 6
   1 5
(2) INFORMATION FOR SEQ ID NO: 26:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:26:
   His Arg Met Arg Met Gly                                                    6
   1               5
(2) INFORMATION FOR SEQ ID NO:27:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:27:
   His Tyr Gly Arg Ser Gly                                                    6
   1               5
(2) INFORMATION FOR SEQ ID NO:28:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:28:
   Ile Met Arg Arg Gly Lys                                                          6
   1               5
(2) INFORMATION FOR SEQ ID NO:29:
   (i) SEQUENCE CHARACTERISTICS:
      (A) LENGTH: 7 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:29:
   Ile Thr Tyr Gly Arg Arg Leu                                                      7
   1               5
(2) INFORMATION FOR SEQ ID NO:30:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:30:
   Lys Phe Thr Arg Ser Gly 6
   1 5
(2) INFORMATION FOR SEQ ID NO:31:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:31:
   Leu Ile Pro Arg Arg Ala 6
   1 5
(2) INFORMATION FOR SEQ ID NO:32:
   (i) SEQUENCE CHARACTERISTICS:
      (A) LENGTH: 7 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:32:
   Met Thr Arg Lys Arg Met Leu 7
   1 5
(2) INFORMATION FOR SEQ ID NO:33:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:33:
   Asn Phe Ala Arg Met Gly                                                  6
   1               5
(2) INFORMATION FOR SEQ ID NO:34:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:34:
   Asn His Leu Arg Lys Ala                                                  6
   1               5
(2) INFORMATION FOR SEQ ID NO:35:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID-NO:35:
   Asn Val Gly Arg Met Gly                                                        6
   1               5
(2) INFORMATION FOR SEQ ID NO:36:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:36:
   Asn Val Ser Arg Arg Gly                                                        6
   1               5
(2) INFORMATION FOR SEQ ID NO:37:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:37:
   Pro Ile Ser Arg Arg Ala 6
   1 5
(2) INFORMATION FOR SEQ ID NO:38:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:38:
   Pro Val Gly Arg Met Gly 6
   1 5
(2) INFORMATION FOR SEQ ID NO:39:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:39:
   Gln Arg Gly Arg Lys Ala 6
   1 5
(2) INFORMATION FOR SEQ ID NO:40:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:40:
   Arg Leu Leu Arg Ser Val                                              6
   1               5
(2) INFORMATION FOR SEQ ID NO:41:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:41:
   Ser Phe Gly Arg Arg His                                              6
   1               5
(2) INFORMATION FOR SEQ ID NO:42:
   (i) SEQUENCE CHARACTERISTICS:
      (A) LENGTH: 7 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:42:
   Ser Leu Arg Gly Arg Ser Leu                                7
   1               5
(2) INFORMATION FOR SEQ ID NO:43:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:43:
   Thr Val Leu Arg Arg Ala                                    6
   1               5
(2) INFORMATION FOR SEQ ID NO:44:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:44:
   Val Ala Arg Arg Val Lys                                    6
   1               5
(2) INFORMATION FOR SEQ ID NO:45:
   (i) SEQUENCE CHARACTERISTICS:
      (A) LENGTH: 6 amino acids
      (B) TYPE: amino acid
      (C) STRANDEDNESS: not. relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:45:
   Val Ile Ala Arg Ser Asn                                    6
   1               5
(2) INFORMATION FOR SEQ ID NO:46:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:46:
   Val Asn Thr Lys Ser Gly                                    6
   1               5
(2) INFORMATION FOR SEQ ID NO:47:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:47:
   Val Arg Ala Arg Gly Ala                                                         6
   1               5
(2) INFORMATION FOR SEQ ID NO:48:
   (i) SEQUENCE CHARACTERISTICS:
      (A) LENGTH: 7 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:48:
   Val Arg Arg Gly Arg Ser Leu                                                     7
   1               5
(2) INFORMATION FOR SEQ ID NO:49:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:49:
   Val Arg Arg Arg Gly Ala                                                          6
   1               5
(2) INFORMATION FOR SEQ ID NO:50:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:50:
   Thr Arg Val Arg Ala Lys                                                          6
   1               5
(2) INFORMATION FOR SEQ ID NO:51:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:51:
   Ser Gly Arg Ser Ala Asn Ala Ile                                                  8
   1               5
(2) INFORMATION FOR SEQ ID NO:52:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:52:
   Ser Gly Arg Asn Ala Gln Val Arg                                         8
   1               5
(2) INFORMATION FOR SEQ ID NO:53:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:53:
   Ser Gly Arg Ser Ala Thr Arg Asp                                         8
   1               5
(2) INFORMATION FOR SEQ ID NO:54:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:54:
   Ser Gly Arg Ser Ala Lys Val Asp                                    8
   1               5
(2) INFORMATION FOR SEQ ID NO:55:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:55:
   Ser Gly Arg Lys Ala Ser Leu Ser                                    8
   1               5
(2) INFORMATION FOR SEQ ID NO:56:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:56:
   Ser Gly Arg Arg Ala Val Ser Asn                                          8
   1               5
(2) INFORMATION FOR SEQ ID NO:57:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:57:
   Ser Gly Arg Ser Ala Val Val Lys                                          8
   1               5
(2) INFORMATION FOR SEQ ID NO:58:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:58:
   Ser Gly Arg Ser Ala Val Val Lys                                          8
   1               5
(2) INFORMATION FOR SEQ ID NO:59:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:59:
   Ala Ile Lys Arg Ser Ala                                                             6
   1               5
(2) INFORMATION FOR SEQ ID NO:60:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:60:
   Gly Arg Arg Gly Asn Arg                                                             6
   1               5
(2) INFORMATION FOR SEQ ID NO:61:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:61:
   Gly Arg Ser Val Asn Asn                                            6
   1               5
(2) INFORMATION FOR SEQ ID NO:62:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:62:
   His Thr Arg Arg Met Lys                                            6
   1               5
(2) INFORMATION FOR SEQ ID NO:63:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:63:
   Ile Ser Thr Ala Arg Met                                            6
   1               5
(2) INFORMATION FOR SEQ ID NO:64:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:64:
   Lys Ala Ala Asp Val Thr                                    6
   1               5
(2) INFORMATION FOR SEQ ID NO:65:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:65:
   Lys Lys Arg Thr Asn Asp                                    6
   1               5
2) INFORMATION FOR SEQ ID NO:66:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:66:
   Lys Met Ser Ala Arg Ile                                              6
   1               5
(2) INFORMATION FOR SEQ ID NO:67:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:67:
   Lys Arg Arg Asp Val Ala                                              6
   1               5
(2) INFORMATION FOR SEQ ID NO:68:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:68:
   Lys Arg Val Ser Lys Asn                                          6
   1               5
(2) INFORMATION FOR SEQ ID NO:69:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:69:
   Lys Ser Ala Asp Ala Ala                                          6
   1               5
(2) INFORMATION FOR SEQ ID NO:70:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:70:
   Arg Ala Ala Ala Met Val 6
   1 5
(2) INFORMATION FOR SEQ ID NO:71:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:71:
   Arg Ala Gly Asn Ile Arg 6
   1 5
(2) INFORMATION FOR SEQ ID NO:72:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO: 72:
   Arg Ala His Arg Asp Asn 6
   1 5
(2) INFORMATION FOR SEQ ID NO:73:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:73:
   Arg Ala Arg Asp Asp Arg                                         6
   1               5
(2) INFORMATION FOR SEQ ID NO:74:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:74:
   Arg Ala Arg His Met Val                                         6
   1               5
(2) INFORMATION FOR SEQ ID NO:75:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:75:
   Arg Ala Arg Ser Pro Arg                                          6
   1               5
(2) INFORMATION FOR SEQ ID NO: 76:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:76:
   Arg Ala Val Gly His Gln                                          6
   1               5
(2) INFORMATION FOR SEQ ID NO :77:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:77:
   Arg Ala Val Val Asp Ser 6
   1 5
(2) INFORMATION FOR SEQ ID NO:78:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:78:
   Arg Gly Gly Lys Gly Pro 6
   1 5
(2) INFORMATION FOR SEQ ID NO:79:
   (i) SEQUENCE CHARACTERISTICS:
      (A) LENGTH: 5 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:79:
   Arg Gly Arg Ser Ala 5
   1 5
(2) INFORMATION FOR SEQ ID NO:80:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:80:
   Arg Gly Val Asp Met Asn                                                    6
   1               5
(2) INFORMATION FOR SEQ ID NO:81:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:81:
   Arg Gly Val Lys Met His                                                    6
   1               5
(2) INFORMATION FOR SEQ ID NO:82:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:82:
   Arg His Arg Ser Asp Ile                                                    6
   1               5
(2) INFORMATION FOR SEQ ID NO:83:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:83:
   Arg Lys Gly Gln Gly Gly                                                    6
   1               5
(2) INFORMATION FOR SEQ ID NO:84:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:84:
   Arg Lys Leu His Met Asn 6
   1 5
(2) INFORMATION FOR SEQ ID NO:85:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:85:
   Arg Lys Met Asp Met Gly 6
   1 5
(2) INFORMATION FOR SEQ ID NO:86:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:86:
   Arg Lys Met Asp Arg Ser 6
   1 5
(2) INFORMATION FOR SEQ ID NO:87:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:87:
   Arg Lys Met Arg Met Gly                                                  6
   1               5
(2) INFORMATION FOR SEQ ID NO:88:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:88:
   Arg Lys Asn Gln Arg Val                                                  6
   1               5
(2) INFORMATION FOR SEQ ID NO:89:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:89:
   Arg Lys Gln Arg Asp Ser                                                    6
   1               5
(2) INFORMATION FOR SEQ ID NO:90:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:90:
   Arg Lys Arg Val Gly Ala                                                    6
   1               5
(2) INFORMATION FOR SEQ ID NO:91:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:91:
   Arg Lys Ser Lys Val Val 6
   1 5
(2) INFORMATION FOR SEQ ID NO:92:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:92:
   Arg Lys Ser Thr Ser Ser 6
   1 5
(2) INFORMATION FOR SEQ ID NO:93:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:93:
   Arg Lys Val Gly Ser Leu 6
   1 5
(2) INFORMATION FOR SEQ ID NO:94:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:94:
   Arg Lys Val Pro Gly Ser                                          6
   1               5
(2) INFORMATION FOR SEQ ID NO:95:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:95:
   Arg Lys Trp Ile Ser Gly                                          6
   1               5
(2) INFORMATION FOR SEQ ID NO:96:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:96:
   Arg Leu Ala Thr Lys Ala                                          6
   1               5
(2) INFORMATION FOR SEQ ID NO:97 :
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:97:
   Arg Met Arg Lys Asn Asp                                          6
   1               5
(2) INFORMATION FOR SEQ ID NO:98:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:98:
   Arg Asn Ala Gln Val Arg 6
   1 5
(2) INFORMATION FOR SEQ ID NO:99:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:99:
   Arg Asn Ala Val Glu Pro 6
   1 5
(2) INFORMATION FOR SEQ ID NO:100:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:100:
   Arg Asn Asp Arg Leu Asn 6
   1 5
(2) INFORMATION FOR SEQ ID NO:101:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:101:
   Arg Asn Gly Lys Ser Arg                                                    6
   1               5
(2) INFORMATION FOR SEQ ID NO:102:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:102:
   Arg Asn Met Pro Leu Leu                                                    6
   1               5
(2) INFORMATION FOR SEQ ID NO:103:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:103:
   Arg Asn Thr Gly Ser His                                                6
   1               5
(2) INFORMATION FOR SEQ ID NO:104:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:104:
   Arg Arg Met Thr Met Gly                                                6
   1               5
(2) INFORMATION FOR SEQ ID NO:105:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:105:
   Arg Arg Arg Leu Asn Met                                    6
   1               5
(2) INFORMATION FOR SEQ ID NO:106:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:106:
   Arg Arg Thr Leu Asp Phe                                    6
   1               5
(2) INFORMATION FOR SEQ ID NO:107 :
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:107:
   Arg Ser Ala Lys Val Asp                                    6
   1               5
(2) INFORMATION FOR SEQ ID NO:108:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:108:
   Arg Ser Ala Asn Ala Ile                                              6
   1               5
(2) INFORMATION FOR SEQ ID NO:109:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:109:
   Arg Ser Ala Thr Arg Asp                                              6
   1               5
(2) INFORMATION FOR SEQ ID NO:110:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:110:
   Arg Ser Ala Val Val Lys                                                    6
   1               5
(2) INFORMATION FOR SEQ ID NO:111:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:111:
   Arg Ser Asp Gln Phe Leu                                                    6
   1               5
(2) INFORMATION FOR SEQ ID NO:112:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:112:
   Arg Ser Asp Asn Pro Asn                                                    6
   1               5
(2) INFORMATION FOR SEQ ID NO:113:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:113:
   Arg Ser Glu Arg Ser Leu                                                      6
   1               5
(2) INFORMATION FOR SEQ ID NO:114:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:114:
   Arg Ser Gly Asp Pro Gly                                                      6
   1               5
(2) INFORMATION FOR SEQ ID NO:115:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:115:
   Arg Ser Gly Asn Thr Thr                                     6
   1               5
(2) INFORMATION FOR SEQ ID NO:116:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:116:
   Arg Ser Gly Asn Met Gly                                     6
   1               5
(2) INFORMATION FOR SEQ ID NO:117:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:117:
   Arg Ser Asn Gly Val Gly                                    6
   1               5
(2) INFORMATION FOR SEQ ID NO:118:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:118:
   Arg Ser Pro Asp Gly Met                                    6
   1               5
(2) INFORMATION FOR SEQ ID NO:119:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:119:
   Arg Ser Arg Arg Leu Pro                                    6
   1               5
(2) INFORMATION FOR SEQ ID NO:120:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:120:
   Arg Ser Arg Val Thr Ser                                              6
   1               5
(2) INFORMATION FOR SEQ ID NO:121:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:121:
   Arg Ser Ser His Ser Ser                                              6
   1               5
(2) INFORMATION FOR SEQ ID NO:122:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:122:
   Arg Ser Ser Gln Ala Ala                                                          6
   1               5
(2) INFORMATION FOR SEQ ID NO:123:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:123:
   Arg Ser Ser Ser Ser His                                                          6
   1               5
(2) INFORMATION FOR SEQ ID NO:124:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:124:
   Arg Ser Ser Ser Thr Val 6
   1 5
(2) INFORMATION FOR SEQ ID NO:125:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE-TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:125:
   Arg Ser Thr Asp Leu Gly 6
   1 5
(2) INFORMATION FOR SEQ ID NO:126:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:126:
   Arg Ser Thr Asn Val Glu 6
   1 5
(2) INFORMATION FOR SEQ ID NO:127:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:127:
   Arg Ser Thr Arg His Lys                                         6
   1               5
(2) INFORMATION FOR SEQ ID NO:128:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:128:
   Arg Ser Tyr Thr Asn Ser                                         6
   1               5
(2) INFORMATION FOR SEQ ID NO:129:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:129:
   Arg Thr Ser Pro Ser Thr                                        6
   1               5
(2) INFORMATION FOR SEQ ID NO:130:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:130:
   Arg Thr Ser Val Asn Leu                                        6
   1               5
(2) INFORMATION FOR SEQ ID NO:131:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:131:
   Ser Gly Arg Ala Arg Gln 6
   1 5
(2) INFORMATION FOR SEQ ID NO:132:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:132:
   Ser Lys Arg Ala Ser Ile 6
   1 5
(2) INFORMATION FOR SEQ ID NO:133:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:133:
   Ser Lys Ser Gly Arg Ser 6
   1 5
(2) INFORMATION FOR SEQ ID NO:134:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:134:
   Ser Gln Thr Cys Val Arg                                  6
   1               5
(2) INFORMATION FOR SEQ ID NO:135:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:135:
   Ser Ser Arg Asn Ala Asp                                  6
   1               5
(2) INFORMATION FOR SEQ ID NO:136:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:136:
   Thr Ala Arg Leu Arg Gly                                                          6
   1               5
(2) INFORMATION FOR SEQ ID NO:137:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:137:
   Thr Ala Arg Ser Asp Asn                                                          6
   1               5
(2) INFORMATION FOR SEQ ID NO:138:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:138:
   Thr Glu Arg Arg Val Arg 6
   1 5
(2) INFORMATION FOR SEQ ID NO:139:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:139:
   Thr Gln Arg Ser Thr Gly 6
   1 5
(2) INFORMATION FOR SEQ ID NO:140:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:140:
   Thr Arg Arg Asp Arg Ile 6
   1 5
(2) INFORMATION FOR SEQ ID NO:141:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:141:
   Thr Ser Arg Met Gly Thr                 6
   1               5
(2) INFORMATION FOR SEQ ID NO:142:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:142:
   Thr Ser Arg Gln Ala Gln                 6
   1               5
(2) INFORMATION FOR SEQ ID NO:143:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO: 143:
   Thr Thr Arg Arg Asn Lys                                                                      6
   1               5
(2) INFORMATION FOR SEQ ID NO:144:
   (i) SEQUENCE CHARACTERISTICS:
      (A) LENTGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:3.44:
(2) INFORMATION FOR SEQ ID NO:145:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:145:
   Val Ala Arg Met Tyr Lys 6
   1 5
(2) INFORMATION FOR SEQ ID NO:146:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:146:
   Val Ser Arg Arg Asn Met 6
   1 5
(2) INFORMATION FOR SEQ ID NO:147:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:147:
   Trp Ser Gly Arg Ser Gly 6
   1 5
(2) INFORMATION FOR SEQ ID NO:148:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:148:
   Arg Ile Ala Arg Arg Ala                                                   6
   1               5
(2) INFORMATION FOR SEQ ID NO:149:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:149:
   Val Ser Ala Arg Met Ala                                                   6
   1               5
(2) INFORMATION FOR SEQ ID NO:150:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:150:
   Val Arg Ala Arg Met Ala                                              6
   1               5
(2) INFORMATION FOR SEQ ID NO:151:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:151:
   Gln Ser Ala Arg Met Ala                                              6
   1               5
(2) INFORMATION FOR SEQ ID NO:152:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:152:
   Gln Arg Ala Arg Met Ala 6
   1 5
(2) INFORMATION FOR SEQ ID NO:153:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:153:
   Ser Gly Arg Ala Arg Gln 6
   1 5
(2) INFORMATION FOR SEQ ID NO:154:
   (i) SEQUENCE CHARACTERISTICS:
      (A) LENGTH: 7 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:154:
   Ser Lys Ser Gly Arg Ser Leu 7
   1 5
(2) INFORMATION FOR SEQ ID NO:155:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:155:
   Ser Ser Arg Asn Ala Asp                                    6
   1               5
(2) INFORMATION FOR SEQ ID NO:156:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:156:
   Thr Ala Arg Leu Arg Gly                                    6
   1               5
(2) INFORMATION FOR SEQ ID NO:157:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:157:
   Thr Ala Arg Ser Asp Asn                                        6
   1               5
(2) INFORMATION FOR SEQ ID NO:158:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:158:
(2) INFORMATION FOR SEQ ID NO:159:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:159:
   Thr Ser Arg Gln Ala Gln                                              6
   1               5
(2) INFORMATION FOR SEQ ID NO:160:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:160:
   Thr Thr Arg Arg Asn Lys                                              6
   1               5
(2) INFORMATION FOR SEQ ID NO:161:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:161:
   Thr Thr Ser Arg Arg Ser                                              6
   1               5
(2) INFORMATION FOR SEQ ID NO:162:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:162:
   Trp Ser Gly Arg Ser Gly                                              6
   1               5
(2) INFORMATION FOR SEQ ID NO:163:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:163:
   Ala Ile Lys Arg Ser Ala                                              6
   1               5
(2) INFORMATION FOR SEQ ID NO:164:
   (i) SEQUENCE CHARACTERISTICS:
      (A) LENGTH: 7 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:164:
   Gly Gly Arg Arg Gly Asn Arg                                        7
   1               5
(2) INFORMATION FOR SEQ ID NO:165:
   (i) SEQUENCE CHARACTERISTICS:
      (A) LENGTH: 7 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:165:
   Gly Gly Arg Ser Val Asn Asn                                        7
   1               5
(2) INFORMATION FOR SEQ ID NO:166:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:166:
   His Thr Arg Arg Met Lys 6
   1 5
(2) INFORMATION FOR SEQ ID NO:167:
   (i) SEQUENCE CHARACTERISTICS:
      (A) LENGTH: 7 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:167:
   Ile Ser Thr Ala Arg Met Leu 7
   1 5
(2) INFORMATION FOR SEQ ID NO:168:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:168:
   Ser Gly Lys Ala Ala Asp Val Thr 8
   1 5
(2) INFORMATION FOR SEQ ID NO:169:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:169:
   Lys Lys Arg Thr Asn Asp                                                6
   1               5
(2) INFORMATION FOR SEQ ID NO:170:
   (i) SEQUENCE CHARACTERISTICS:
      (A) LENGTH: 7 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:170:
   Lys Met Ser Ala Arg Ile Leu                                            7
   1               5
(2) INFORMATION FOR SEQ ID NO:171:
   (i) SEQUENCE CHARACTERISTICS:
      (A) LENGTH: 7 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:171:
   Gly Lys Arg Arg Asp Val Ala                                           7
   1               5
(2) INFORMATION FOR SEQ ID NO:172:
   (i) SEQUENCE CHARACTERISTICS:
      (A) LENGTH: 7 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:172:
   Gly Lys Arg Val Ser Lys Asn                                           7
   1               5
(2) INFORMATION FOR SEQ ID NO:173:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:173:
   Ser Gly Lys Ser Ala Asp Ala Ala 8
   1 5
(2) INFORMATION FOR SEQ ID NO:174:
   (i) SEQUENCE CHARACTERISTICS:
      (A) LENGTH: 7 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:174:
   Ser Gly Arg Ala Ala Ala Met 7
   1 5
(2) INFORMATION FOR SEQ ID NO:175:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:175:
   Ser Gly Arg Ala Gly Asn Ile Arg 8
   1 5
(2) INFORMATION FOR SEQ ID NO:176:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:176:
   Ser Gly Arg Ala His Arg Asp Asn                                8
   1               5
(2) INFORMATION FOR SEQ ID NO:177:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO: 177:
   Ser Gly Arg Ala Arg Asp Asp Arg                                8
   1               5
(2) INFORMATION FOR SEQ ID NO:178:
   (i) SEQUENCE CHARACTERISTICS:
      (A) LENGTH: 7 amino acids
      (B) TYPE: amino acid
      (C) STRANDEDNESS: not: relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:178:
   Ser Gly Arg Ala Arg His Met                                     7
   1               5
(2) INFORMATION FOR SEQ ID NO:179:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:179:
   Ser Gly Arg Ala Arg Ser Pro Arg                                 8
   1               5
(2) INFORMATION FOR SEQ ID NO:180:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:180:
   Ser Gly Arg Ala Val Gly His Gln                                 8
   1               5
(2) INFORMATION FOR SEQ ID NO:181:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:181:
   Ser Gly Arg Ala Val Val Asp Ser                    8
   1               5
(2) INFORMATION FOR SEQ ID NO:182:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:182:
   Ser Gly Arg Gly Gly Lys Gly Pro                    8
   1               5
(2) INFORMATION FOR SEQ ID NO:183:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:183:
   Ser Gly Arg Gly Arg Ser Ala Val                                              8
   1               5
(2) INFORMATION FOR SEQ ID NO:184:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:184:
   Ser Gly Arg Gly Val Asp Met Asn                                              8
   1               5
(2) INFORMATION FOR SEQ ID NO:185:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:185:
   Ser Gly Arg Gly Val Lys Met His 8
   1 5
(2) INFORMATION FOR SEQ ID NO:186:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:186:
   Ser Gly Arg His Arg Ser Asp Ile 8
   1 5
(2) INFORMATION FOR SEQ ID NO:187:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:187:
   Ser Gly Arg Lys Gly Gln Gly Gly 8
   1 5
(2) INFORMATION FOR SEQ ID NO:188:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:188:
   Ser Gly Arg Lys Leu His Met Asn                                  8
   1               5
(2) INFORMATION FOR SEQ ID NO:189:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:189:
   Ser Gly Arg Lys Met Asp Met Gly                                  8
   1               5
(2) INFORMATION FOR SEQ ID NO: 190:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:190:
   Ser Gly Arg Lys Met Asp Arg Ser                                        8
   1               5
(2) INFORMATION FOR SEQ ID NO:191:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:191:
   Ser Gly Arg Lys Met Arg Met Gly                                        8
   1               5
(2) INFORMATION FOR SEQ ID NO:192:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:192:
   Ser Gly Arg Lys Asn Gln Arg Val 8
   1 5
(2) INFORMATION FOR SEQ ID NO:193:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:193:
   Ser Gly Arg Lys Gln Arg Asp Ser 8
   1 5
(2) INFORMATION FOR SEQ ID NO:194:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:194:
   Ser Gly Arg Lys Arg Val Gly Ala 8
   1 5
(2) INFORMATION FOR SEQ ID NO:195:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:195:
   Ser Gly Arg Lys Ser Lys Val Val                                  8
   1               5
(2) INFORMATION FOR SEQ ID NO:196:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:196:
   Ser Gly Arg Lys Ser Thr Ser Ser                                  8
   1               5
(2) INFORMATION FOR SEQ ID NO:197:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:197:
   Ser Gly Arg Lys Val Gly Ser Leu                                   8
   1               5
(2) INFORMATION FOR SEQ ID NO: 198:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:198:
   Ser Gly Arg Lys Val Pro Gly Ser                                   8
   1               5
(2) INFORMATION FOR SEQ ID NO:199:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:199:
   Ser Gly Arg Lys Trp Ile Ser Gly 8
   1 5
(2) INFORMATION FOR SEQ ID NO:200:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:200:
   Ser Gly Arg Leu Ala Thr Lys Ala 8
   1 5
(2) INFORMATION FOR SEQ ID NO:201:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:201:
   Ser Gly Arg Met Arg Lys Asn Asp 8
   1 5
(2) INFORMATION FOR SEQ ID NO:202:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:202:
   Ser Gly Arg Asn Ala Val Glu Pro                                      8
   1               5
(2) INFORMATION FOR SEQ ID NO:203:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:203:
   Ser Gly Arg Asn Asp Arg Leu Asn                                      8
   1               5
(2) INFORMATION FOR SEQ ID NO:204:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:204:
   Ser Gly Arg Asn Gly Lys Ser Arg                                          8
   1               5
(2) INFORMATION FOR SEQ ID NO:205:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:205:
   Ser Gly Arg Asn Met Pro Leu Leu                                          8
   1               5
(2) INFORMATION FOR SEQ ID NO:206:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:206:
   Ser Gly Arg Asn Thr Gly Ser His                                          8
   1               5
(2) INFORMATION FOR SEQ ID NO:207:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:207:
   Ser Gly Arg Arg Met Thr Met Gly          8
   1               5
(2) INFORMATION FOR SEQ ID NO:208:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:208:
   Ser Gly Arg Arg Arg Leu Asn Met          8
   1               5
(2) INFORMATION FOR SEQ ID NO: 209:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:209:
   Ser Gly Arg Arg Thr Leu Asp Phe                                    8
   1               5
(2) INFORMATION FOR SEQ ID NO-.210:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:210:
(2) INFORMATION FOR SEQ ID NO: 211:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:211:
   Ser Gly Arg Ser Asp Asn Pro Asn 8
   1 5
(2) INFORMATION FOR SEQ ID NO:212:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE-DESCRIPTION: SEQ ID NO:212:
(2) INFORMATION FOR SEQ ID NO:213:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:213:
   Ser Gly Arg Ser Gly Asp Pro Gly 8
   1 5
(2) INFORMATION FOR SEQ ID NO:214:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:214:
   Ser Gly Arg Ser Gly Asn Thr Thr          8
   1               5
(2) INFORMATION FOR SEQ ID NO:215:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:215:
   Ser Gly Arg Ser Gly Asn Met Gly          8
   1               5
(2) INFORMATION FOR SEQ ID NO:216:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:216:
   Ser Gly Arg Ser Asn Gly Val Gly                                    8
   1               5
(2) INFORMATION FOR SEQ ID NO:217:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:217:
   Ser Gly Arg Ser Pro Asp Gly Met                                    8
   1               5
(2) INFORMATION FOR SEQ ID NO:218:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:218:
   Ser Gly Arg Ser Arg Arg Leu Pro 8
   1 5
(2) INFORMATION FOR SEQ ID NO:219:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:219:
   Ser Gly Arg Ser Arg Val Thr Ser 8
   1 5
(2) INFORMATION FOR SEQ ID NO:220:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:220:
   Ser Gly Arg Ser Ser His Ser Ser 8
   1 5
(2) INFORMATION FOR SEQ ID NO:221:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:221:
   Ser Gly Arg Ser Ser Gln Ala Ala          8
   1               5
(2) INFORMATION FOR SEQ ID NO:222:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:222:
   Ser Gly Arg Ser Ser Ser Thr Val          8
   1               5
(2) INFORMATION FOR SEQ ID NO:223:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:223:
   Ser Gly Arg Ser Thr Asp Leu Gly          8
   1               5
(2) INFORMATION FOR SEQ ID NO:224:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:224:
   Ser Gly Arg Ser Thr Asn Val Glu          8
   1               5
(2) INFORMATION FOR SEQ ID NO:225:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:225:
   Ser Gly Arg Ser Thr Arg His Lys          8
   1               5
(2) INFORMATION FOR SEQ ID NO:226:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:226:
   Ser Gly Arg Ser Tyr Thr Asn Ser          8
   1               5
(2) INFORMATION FOR SEQ ID NO:227:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE-TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:227:
   Ser Gly Arg Thr Ser Pro Ser Thr          8
   1               5
(2) INFORMATION FOR SEQ ID NO:228:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:228:
   Ser Gly Arg Thr Ser Val Asn Leu                                    8
   1               5
(2) INFORMATION FOR SEQ ID NO:229:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:229:
   Ser Lys Arg Ala Ser Ile                                            6
   1               5
(2) INFORMATION FOR SEQ ID NO:230:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:230:
   Ser Gln Thr Cys Val Arg Leu Val                                      8
   1               5
(2) INFORMATION FOR SEQ ID NO:231:
   (i) SEQUENCE CHARACTERISTICS:
      (A) LENGTH: 8 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv), ANTI-SENSE: NO
   (vi) ORIGINAL SOURCE:
      (A) ORGANISM: Homo sapiens
   (xi) SEQUENCE DESCRIPTION: SEQ ID NO:231:
   Thr Glu Arg Arg Val Arg Leu Val                                      8
   1               5
(2) INFORMATION FOR SEQ ID NO:232:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:232:
   Thr Gln Arg Ser Thr Gly                                              6
   1               5
(2) INFORMATION FOR SEQ ID NO:233:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:233:
   Thr Arg Arg Asp Arg Ile                                     6
   1               5
(2) INFORMATION FOR SEQ ID NO:234:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:234:
   Val Ala Arg Met Thr Lys                                     6
   1               5
(2) INFORMATION FOR SEQ ID NO:235:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:235:
   Val Ser Arg Arg Asn Met                                              6
   1               5
(2) INFORMATION FOR SEQ ID NO:236:
   (i) SEQUENCE CHARACTERISTICS:
      (A) LENGTH: 13 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:236:
   Leu Gly Gly Tyr Gly Arg Ser Ala Asn Ala Ile Leu Glu                 13
   1               5                   10
(2) INFORMATION FOR SEQ ID NO:237:
   (i) SEQUENCE CHARACTERISTICS:
      (A) LENGTH: 13 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:237:
   Leu Gly Gly Arg Gly Arg Ser Ala Asn Ala Ile Leu Glu 13
   1 5 10
(2) INFORMATION FOR SEQ ID NO:238:
   (i) SEQUENCE CHARACTERISTICS:
      (A) LENGTH: 13 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:238:
   Leu Gly Gln Arg Gly Arg Ser Ala Asn Ala Ile Leu Glu 13
   1 5 10
(2) INFORMATION FOR SEQ ID NO:239:
   (i) SEQUENCE CHARACTERISTICS:
      (A) LENGTH: 6 amino acids
      (B) TYPE: amino acid
      (C) STRANDEDNESS: not relevant
      (D) TOPOLOGY: linear
   (ii) MOLECULE TYPE: peptide
   (iii) HYPOTHETICAL: NO
   (iv) ANTI-SENSE: NO
   (vi) ORIGINAL SOURCE:
      (A) ORGANISM: Homo sapiens
   (xi) SEQUENCE DESCRIPTION: SEQ ID NO:239:
   Gly Ser Gly Lys Ser Ala 6
   1 5

## Description

### Reference to Related Application

This application claims the benefit of U.S. Provisional Application S.N. 60/019,495, filed June 10, 1996.

### Governmental Rights

This invention was made with governmental support from the United States Government, National Institutes of Health, Grants HL52475 and HL31950; the United States Government has certain rights in the invention.

### Field of the Invention

The invention relates to methods for elucidating specificity differences between closely related enzymes and making enzyme inhibitors based on those differences.

### Background of the Invention

The chymotrypsin family of serine proteases has evolved to include members with intimately related substrate specificities (Perona, J.J. & Craik, C.S., Structural basis of substrate specificity in the serine proteases, Protein Science **4**: 337-360, 1995). Two of these enzymes, t-PA and u-PA, were chosen to test the hypothesis that small molecule libraries could be used to identify substrates that discriminate between closely related enzymes. These two proteases possess an extremely high degree of structural similarity (Spraggon G, Phillips C, Nowak UK, et al. The crystal structure of the catalytic domain of human urokinase-type plasminogen activator, Structure **3**:681-691, 1995), share the same primary physiological substrate (plasminogen) and inhibitors (plasminogen activator inhibitor types 1 and 2), exhibit remarkably stringent substrate specificity, and play key roles in critical biological processes. Plasminogen activator inhibitors are examples of the class of molecules known as serpins (serine protease inhibitors) (Lawrence, D.A. and Ginsburg, D., in Molecular Basis of Thrombosis and Hemostasis, K. A. High, H. R. Roberts, Eds., Marcel Dekker, New York, 1995, pp. 517-543).

In general, proteases and their inhibitors, such as serine proteases and serpins, are involved in numerous biological processes in addition to fibrinolysis, such as ovulation, fertilization, embryogenesis, angiogenesis, infection and inflammation. See, generally, Katunuma, N., et al., editors, Biological Functions of Proteases and Inhibitors, Karger, Tokyo, 1994; Troll, W., and Kennedy, A.R., editors, Protease Inhibitors as Cancer Chemopreventive Agents, Plenum Press, New York, 1993; Gettins, P.G.W., et al., editors, Serpins: Structure, Function and Biology, Chapman and Hall, New York, 1996; Sandler, M., and Smith, H.J., editors, Design of Enzyme Inhibitors as Drugs, Oxford University Press, New York, 1989). One particularly important use of protease inhibitors is in the treatment of HIV infection and AIDS (Huff, J.R., and Darke, P.L., Inhibition of HIV protease: A strategy to the treatment of AIDS, in Mohan, P., and Baba, M., editors, Anti-AIDS Drug Development, Harwood Academic Publishers, Chur, 1995)

Local activation and aggregation of platelets, followed by initiation of the blood coagulation cascade (collectively part of what is referred to as the hemostatic system), assure that a fibrin clot will form rapidly in response to vascular injury (Roberts, H. R., and Tabares, A. H. (1995) in Molecular Basis of Thrombosis and Hemostasis, K. A. High, H. R. Roberts, Eds., Marcel Dekker, New York, N.Y., 1995, pp. 35-50). The presence of this clot, however, must be transient if the damaged tissue is to be remodeled and normal blood flow restored. The fibrinolytic system, which accomplishes the enzymatic degradation of fibrin, is therefore an essential component of the hemostatic system. The ultimate product of the fibrinolytic system is plasmin, a chymotrypsin family enzyme with relatively broad, trypsin-like primary specificity that is directly responsible for the efficient degradation of a fibrin clot (Castellino, F. J. (1995) in Molecular Basis of Thrombosis and Hemostasis, K. A. High, H. R. Roberts, Eds., Marcel Dekker, New York, 1995, pp. 495-515). Production of this mature proteolytic enzyme from the inactive precursor, or zymogen, plasminogen is the rate limiting step in the fibrinolytic cascade (Collen, D., and Lijnen, H. R. (1991) Blood 78, 3114-3124). Catalysis of this key regulatory reaction is tightly controlled in vivo and is mediated by two enzymes present in human plasma, u-PA and t-PA.

u-PA and t-PA are very closely related members of the chymotrypsin gene family. These two proteases possess extremely high structural similarity (Spraggon, G., et al., (1995) Structure **3**: 681-691; Lamba, B., et al., (1996) J. Mol. Biol. **258:** 117-135), share the same primary physiological substrate (plasminogen) and inhibitor (plasminogen activator inhibitor type 1, PAI-1) (Lawrence and Ginsburg, 1995), and, unlike plasmin, exhibit remarkably stringent substrate specificity.

In spite of their striking similarities, the physiological roles of t-PA and u-PA are distinct (Carmeliet, P.,et al. (1994) Nature **368**: 419-424; Carmeliet, P., and Collen, D. (1996) Fibrinolysis **10**: 195-213). Many studies (5, 6, 12-18) suggest selective inhibition of either enzyme should have beneficial therapeutic effects. Mice lacking t-PA, for example, are resistant to specific excitotoxins which cause extensive neurodegeneration in wild type mice, and mice lacking u-PA exhibit defects in the proliferation and/or migration of smooth muscle cells in a model of restenosis following vascular injury.

Either increased levels of protease inhibitors, such as PAI-1, or decreased levels may be associated with diseases. Increased levels if PAI-1 in the circulation are associated with thrombotic disease, including myocardial infarction and deep vein thrombosis, and reduced post-operative fibrinolytic activity (Lawrence and Ginsburg (1995) page 526). Conditions in which completely or partially reduced levels of PAI-1 are found include bleeding conditions (Schleef, R.R., et al., J. Clin. Invest. **83**: 1747-1752 (1989); Fay, W.P., et al, N. Eng. J. Med. **327**: 1729-1733 (1992); Liu, Y.-X., et al., Eur. J. Biochem. **195**: 54-555, 1991).

A large body of experimental evidence from studies involving both model systems and human patients suggests that u-PA may play an important role in tumor biology and provides a compelling rationale to pursue the development of u-PA inhibitors. For example, anti-u-PA antibodies inhibit metastasis of HEp3 human carcinoma cells to chick embryo lymph nodes, heart, and lung (Ossowski, L., and Reich, E. (1983) Cell **35**: 611-619), and similar studies demonstrated that these antibodies inhibit lung metastasis in mice following injection of B16 melanoma cells into the tail vein (Hearing, V. J., et al., (1988) Cancer Res. **48**: 1270-1278). Anti-u-PA antibodies also inhibit both local invasiveness and lung metastasis in nude mice bearing subcutaneous MDA-MB-231 breast carcinoma tumors. In addition, a recent study indicated that u-PA deficient mice are resistant to the induction and/or progression of several tumor types in a two stage, chemical carcinogenesis model. Finally, high levels of tumor-associated u-PA correlate strongly with both a shortened disease-free interval and poor survival in several different human cancers (Duffy, M. J., et al., (1988) Cancer **62**: 531-533; Janicke, F., et al., (1990) Fibrinolysis **4**: 69-78; Duffy, M. J. (1993) Fibrinolysis **7**: 295-302).

Because mice lacking either u-PA or t-PA do not develop thrombotic disorders, selective inhibition of either of these two enzymes seems unlikely to create thrombotic complications in vivo. On the other hand, mice lacking both u-PA and t-PA suffer severe thrombosis in many organs and tissues, resulting in a significantly reduced life expectancy. Nonselective inhibition of these two enzymes, therefore, seems almost certain to produce catastrophic consequences in the clinical setting. Consequently, significant interest exists in the development of inhibitors that are stringently specific for either t-PA or u-PA, which are expected to facilitate a detailed investigation of the precise roles of the two enzymes in several important pathological processes and may aid the development of novel therapeutic agents to combat these processes. Rational design of these selective inhibitors is greatly complicated, however, by the absence of obvious "lead compounds"; both their primary physiological substrate and inhibitors fail to discriminate between the two closely related proteases.

Combinatorial libraries provide a convenient means for screening a very large number of compounds. In general, combinatorial libraries provide a large number (10⁴ - 10⁸) of variants of small molecules such as peptides (Lam, K.S., Synthetic peptide libraries, in Meyers, R.A., Molecular Biology and Biotechnology. A Comprehensive Desk Reference, VCH Publishers, New York, 1995, pages 880-883). Combinatorial peptide libraries are large collections of different peptides, with many different possible combinations of amino acids joined together. The length of the peptides in the library can be chosen to suit the particular application.

The different molecules in a combinatorial library can be provided with a tag, such as an epitope recognized by an antibody, that may be used for identification and manipulation. Libraries in general can be constructed by synthesis of the different molecules on a substrate, or by a biological method, generally involving bacteriophages and bacteria.

Substrate bacteriophage display libraries have been used to elucidate optimal sub-site occupancy for substrates of t-PA and to isolate peptide substrates that were cleaved as much as 5300 times more efficiently by t-PA than peptides which contained the primary sequence of the actual target site present in plasminogen (Ding, C., et al., 1995). These selected substrates, however, were also efficiently cleaved by u-PA and therefore showed less than an order of magnitude preference for cleavage by t-PA compared to u-PA.

What is needed is a method for identifying substrates that show between about 10 fold to about 1,000 fold selectivity for one enzyme over another.

### Summary of the Invention

The rational design of small molecule inhibitors as therapeutic agents is often complicated by the need to discriminate between binding to closely related enzymes. Appropriate selections of substrate phage can achieve this discrimination. Substrate subtraction libraries of the present invention provide substrates that can distinguish between any two distinct proteases. Multiple proteases can be used in the subtraction step to achieve even greater specificity. Moreover, both substrate and substrate subtraction libraries can be prepared as described herein for any enzymes that can use peptides or proteins as substrates.

The present invention creates substrate subtraction libraries that are useful in the identification of highly selective substrates for specific proteins and enzymes. The present invention is particularly useful in the identification of highly selective substrates for closely-related enzymes. Indeed, it is possible to prepare substrate subtraction libraries for any enzymes that use peptides or proteins as substrates. These techniques can be easily adapted to protein kineses, for example, by using antibodies against phosphoserine, phosphothreonine, or phosphotyrosine during the selection of substrate phage. Consequently, the construction and characterization of substrate and substrate subtraction libraries make substantial contributions to the rational design of highly specific, small molecule inhibitors of selected enzymes, a problem of paramount importance during the development of new therapeutic agents, and to provide key insights into the molecular determinants of specificity for a variety of important enzymes.

In one embodiment, the present invention provides a method for identifying the amino acid sequence of a peptide that is preferentially a substrate for a second enzyme. A combinatorial library having components that present corresponding peptides having random amino acid sequences of a chosen length is provided. The components of the combinatorial library are contacted with the first enzyme and the library separated into two portions, one that has peptides that were modified by the first enzyme and one that has peptides that were not. One or both portions of combinatorial library are contacted with the second enzyme, and the components of the combinatorial library that present corresponding peptides that were modified by contact with the one enzyme but not substantially modified by the other enzyme are identified. The sequences of the corresponding peptides that were modified by the one enzyme can then be determined. The process described is called "substrate subtraction screening" and the combinatorial libraries produced are called "substrate subtraction libraries." The first enzyme and second enzyme are generally in the same broad class of enzymes, e.g., proteases, kinases, phosphatases, and the like. A suitable combinatorial library is a bacteriophage display library.

Another embodiment is a compound comprising the animo acid sequence determined by the method of substrate subtraction screening described above. Such compounds are useful as enzyme inhibitors.

Stringently specific small molecule inhibitors can not only be used to assess the individual roles of t-PA and u-PA during a wide variety of biological and pathological processes but also can provide important therapeutic benefits. Selective inhibition of u-PA can antagonize invasion, metastasis, and angiogenesis of specific tumors (Danø K., et al., Plasminogen activators, tissue degradation, and cancer. Adv. Cancer Res. 1985;44:139-266; Min, H.Y., et al., Urokinase receptor antagonists inhibit angiogenesis and primary tumor growth in syngeneic mice. Cancer Res. (1996);in press; Ossowski, L., Plasminogen activator dependent pathways in the dissemination of human tumor cells in the chick embryo. Cell 1988;52:321-328) as well as vascular re-stenosis following invasive procedures such as angioplasty (Carmeliet P, et al. Physiological consequences of loss of plasminogen activator gene function in mice. Nature 1994;368:419-424). Selective inhibition of t-PA can prevent specific types of neural degeneration (Strickland DK. Excitotoxin-induced neuronal degeneration and seizure are mediated by tissue plasminogen activator. Nature 1995;377:340-344).

In preferred embodiments, the sequence determined by the method of substrate subtraction screening is incorporated in the construction of recombinant protease inhibitors, such as variants of PAI-1. In therapeutic embodiments, recombinant protease inhibitors, such as variants of PAI-1 are administered to a patient in an amount from about 0.003 to about 20 micrograms per kilogram body weight per day.

In another embodiment, antibodies to peptides identified using substrate subtraction libraries are also useful as an assay kit and method for detecting the level of active protease inhibitors. The antibodies may be used to assay the level of protease inhibitors, such as PAI-1, in a patient. The level of protease inhibitors, such as PAI-1, in a patient is a disease marker that is useful for predicting the development of a condition, identifying patients with the condition, predicting outcome of the condition, aiding timing and targeting of therapeutic interventions, and determining the pathogenesis of the condition in patients. Conditions in which the level of protease inhibitors, such as PAI-1, is a useful marker are bleeding conditions characterized by an inability to produce PAI-1 or a lack of active PAI-1. Antibodies to the reactive site of a serpin protease inhibitor such as PAI-1 would be useful for distinguishing between active and latent forms of the protease inhibitor.

In another embodiment, antibodies to peptides identified using substrate subtraction libraries are also useful to identify novel active protease inhibitors. The antibodies may be used to identify molecules having exposed sequences similar to the sequences of peptides identified using substrate subtraction libraries. This embodiment also is useful for screening naturally occurring compounds for protease inhibitor activity in the process of drug discovery.

In a further embodiment, antibodies to the identified peptides can be used for affinity purification of the peptides identified by the present invention. The peptides to be purified can be in a mixture of peptides or can be peptides produced by recombinant techniques.

Suitable antibodies comprise immunoglobulin molecules and immunologically active portions of immunoglobulin molecules, i.e., molecules that contain an antibody combining site or paratope. Exemplary antibody molecules are intact immunoglobulin molecules, substantially intact immunoglobulin molecules and those portions of an immunoglobulin molecule that contain the paratope, including those portions known in the art as Fab, Fab' and F(ab')₂.

### Brief Description of the Drawings

In the Drawings,
FIGURE 1 is a diagram depicting an outline of one protocol used to create substrate subtraction libraries, where the gene III fusion protein, phage, monoclonal antibodies, and immobilized protein A are not drawn to scale;
FIGURE 2 is a diagram depicting an outline of another protocol used to create substrate subtraction libraries;
FIGURE 3 is a representation of the results of a functional analysis of individual control or substrate phage stocks using a dot blot assay; and
FIGURE 4 is a representation of the results of a functional analysis of specific cleavage of a fusion protein by t-PA.

### Detailed Description of Preferred Embodiments

The disclosed methods are useful general in the design of specific inhibitors of various proteins and enzymes. The method can be applied to other proteases, and to other classes of enzymes, including kinases and phosphatases. Thus, the disclosures relating to u-PA and t-PA should be considered exemplary and not limiting.

Examples of other suitable enzyme systems include proteases, including other serine proteases, as well as kinases and phosphatases.

The present invention relates methods of identifying those peptides that are selectively reactive between a first enzyme and a second enzyme. A combinatorial library displaying different peptides is provided. This combinatorial library can be made, for example, by random mutation of bacteriophages displaying peptide sequences. The phage express the peptide sequences externally and, after reaction with the enzymes, the desired phage can be enriched. Alternatively, the combinatorial library can include an array of peptide substrates whose amino acid sequences are known by their location on the substrate. The production of such library arrays on substrates is well known in the art. See, for example, Meyers, Molecular Biology and Biotechnology: A Comprehensive Desk Reference, VCM Publishers, New York 1995, pages 880-883. The use of a phage library is preferred because it is generally able to a greater range of different sequences, usually on the order of 10⁸ different amino acid sequences.

The combinatorial library is contacted with the first enzyme to permit the first enzyme to modify some of the components of the combinatorial library. Those components which are modified by the first enzyme are then separated from the components of the library that are substantially unmodified by the first enzyme.

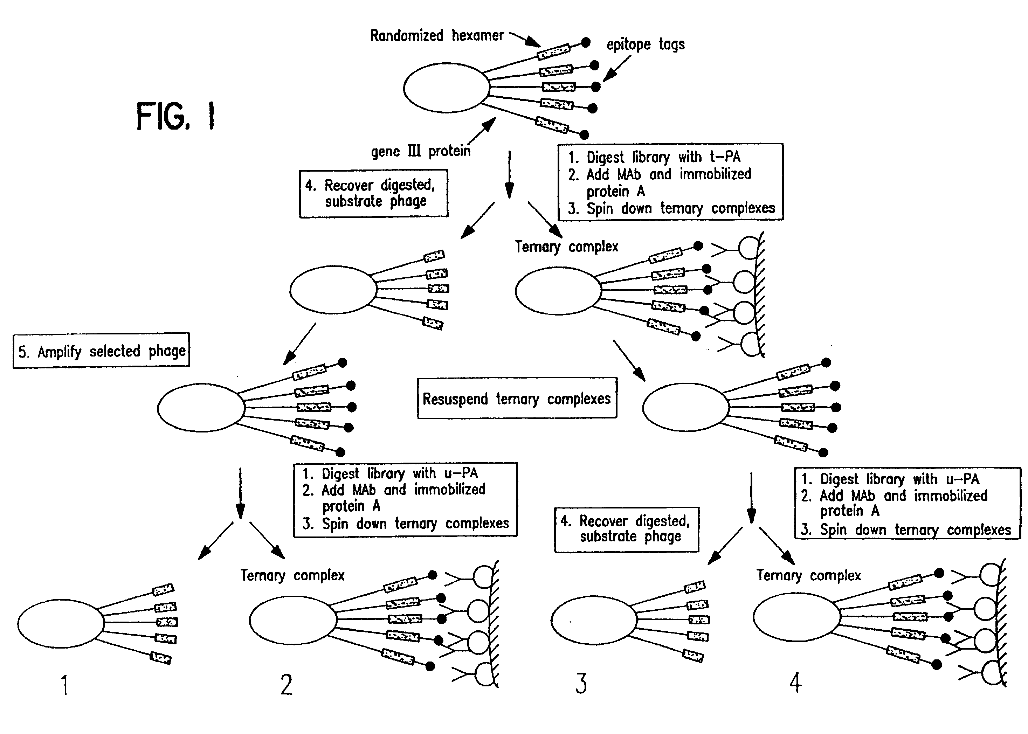

At that point, either one of two steps is then taken. The modified portion is contacted with the second enzyme or the unmodified portion is contacted with the second enzyme. It is then possible to identify at least some of the components of the combinatorial library that are modified by one enzyme but substantially unmodified by the other. A working example showing these pathways is demonstrated in FIGURE 1. The example of phage displaying hexamer peptides with epitope tags on the end of the peptide is shown. The phage library is then allowed to contact the t-PA enzyme to digest the peptides. This results in the separation into two portions, those components which were modified by the t-PA are shown on the left and those not modified by the t-PA are shown on the right. As shown, separation is accomplished by immobilizing with monoclonal antibodies.

The phage that were modified by t-PA is then amplified to produce phage displaying the entire peptide and epitope tabs. As shown on the right, the phage were then digested with u-PA and the phage which were modified were separated from unmodified phage by the use of monoclonal antibodies. This results in a first population of phage expressing peptides that react with both t-PA and u-PA. It also results in a second population that reacts with t-PA but not u-PA. It is the second population that is of interest in the present invention. This population can be resuspended and amplified.

Alternatively shown on the left side of FIGURE 1, the population of phage that was unmodified by t-PA are resuspended and contacted with u-PA. After digestion, monoclonal antibodies are used as before. This results in two more populations, a third population which includes phage expressing peptides that react with u-PA but not t-PA, and a fourth population which includes phage that did not react with either u-PA or t-PA. It is the third population which is also of interest in the present invention. This population can then be amplified.

As can be seen, both of these routes allow the identification of the some of the components of the combinatorial library that are modified by one enzyme but not modified by the other. As shown in FIGURE 1, these are the third and fourth populations. The entire procedure or individual screening steps can be repeated one or more times to increase the selectivity.

At least some of the components that are modified by one enzyme but are not modified by the other enzyme are then identified. In the case of the phage library, phage in the second and third populations are identified in this manner. The phage can then be grown in culture allowing the identification to also include determining the amino acid sequence of at least one of the displayed peptides. The resulting peptides have a selectivity preferably of at least 10 fold, and more preferably of at least 50 fold, for the desired enzyme compound to the undesired enzyme. The enzymes are preferably both proteases such as kinases and phosphatases.

In the case of library arrays on substrates, portions of the library, such as the use of two identical arrays, are individually reacted with each of the enzymes. The location of peptide cleaving is determined, such as by use of a cysteine residue or an epitope tag and labeled antibodies on each array and the results compared to determine which peptides react with one enzyme but not the other.

The amino acid sequence or sequences that are determined can then be used to make peptides having these sequences. These peptides can be used to prepare antibodies as is known in the art and used to purify recombinant peptides or identify naturally occurring protease inhibitors which are immunoreactive with the antibodies so produced. The antibodies are also used for diagnostic assays which can distinguish between active and latent forms of the protease inhibitors.

The amino acid sequences determined can be used to engineer protease inhibitors. For example, the amino acid sequence determined to be highly selective toward to the second enzyme can be used in the design of an inhibitor for the second enzyme which has a low reactivity with the first enzyme. This allows the treatment of medical conditions where targeting of inhibition of the second enzyme is useful, while inhibiting the first enzyme is not desired.

Such inhibitors would have a structure corresponding to naturally occurring enzyme inhibitors. The amino acid sequence of such inhibitors would be modified to include the amino acid sequence taught by the method of this invention. Alternatively, substrates including amino acid sequences as taught by the present invention are modified to create inhibitors.

The amino acid sequence, together with the other coding for the inhibitor is then coded on a plasmid or other DNA vector for introduction into a prokaryotic or eukaryotic cell as is well known in the art. Such cells will produce the desired enzyme inhibitor which can be purified using the antibodies discussed above.

A substrate subtraction combinatorial library can also be produced. The combinatorial library is contacted with the first enzyme to modify some of the components of the combinatorial library. The phage can then be separated by solid phase or precipitation as known in the art with either population serving as a substrate substraction library. The example of the first enzyme will be used.

Such a substrate subtraction library substantially lacks peptides that are effective substrates for the first enzyme, meaning that the peptides have a reactivity of less than about 10 percent of the best naturally occurring molecule that the first enzyme reacts with. For example, in the case of u-PA as shown in Table 5 below, the native or wild type PAI-1 has a rate constant of 1.9 X 10⁷M⁻¹s⁻¹ while the selected PPAI-1/P3R has a rate constant of 1.0 X 10⁵ meaning that the reactivity is less than 1 percent. Table 8 also makes similar comparisons with the wild type. The peptides in the combinatorial library preferably have a k_{cat}/Kₘ ratio of less than about 500 M⁻¹s⁻¹ and preferably a k_{cat}/Kₘ ratio of less than about 100 M⁻¹s⁻¹.

While the removal of the peptides that are effective substrates for the first enzyme is preferred for preparing a substrate subtraction library, it is not necessary for practicing the invention. Depending on the enzyme involved, the method for the present invention can be practiced with peptides having a reactivity even greater than 10% when compared to the naturally occurring molecule.

Herein described is also the therapeutic treatment of a patient. This treatment can take two general forms. The determined peptide itself can be administered to the patient to in effect overload the patient's enzymes thereby creating an inhibitory effect. In such cases, the preferred administration rates of the peptides, such as t-PA and u-PA, are from about 0.1 micrograms/kg to about 50 micrograms/kg.

Alternatively, enzyme inhibitors made according to the present invention can also be administered to the patient. These inhibitors directly inhibit the activity of the enzymes. In the case of u-PA and t-PA, the preferred administration rates are from about 0.003 micrograms/kg to about 20 micrograms/kg.

Some of the diseases that can be effectively treated are serpin deficiencies such as pulmonary emphysema, associated with deficiencies of α₁-proteinase inhibitor, antithrombin deficiency, hereditary angioedema associated with deficiencies of C1- inhibitor, bleeding disorders associated with deficiencies in α-antiplasmin or PAI-1 (Gettins et al. 1996). Serpins have also been implicated in several forms of cancer, including squamous cell carcinoma (Gettins, et al. 1996). In each case, a physiologically effective amount of the peptide or the enzyme inhibitor is administered. Examples of specific peptides are discussed below.

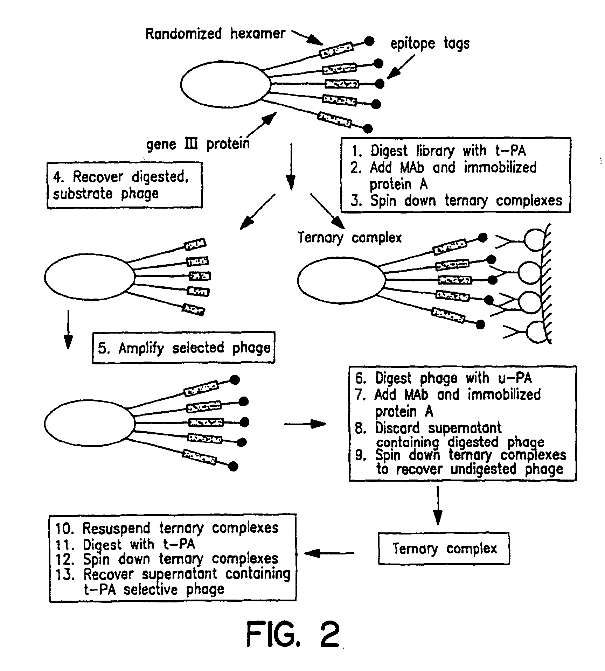

Referring to FIGURE 2, the specific example of phage having hexamer peptides and epitope tags are disclosed. This example pre-processes the library to enhance selectivity. The phage library is then contacted with the t-PA enzyme and digested. Monoclonal antibodies to the epitope tags are then added to separate cleaved and uncleaved expressed peptides. The phage having cleaved peptides are then selected and amplified. The amplified phage are then again digested with u-PA. Monoclonal antibody to the epitope tags is again used to immobilize the phage having peptides that were not modified by the u-PA. The undigested phage are then recovered and resuspended. They are again digested with t-PA and the unreacted phage are again separated using monoclonal antibodies. The phage which are again reacted with t-PA are then identified in the supernatant.

### EXAMPLE 1:

### Preparation and Use of Substrate Subtraction Libraries

### Reagents.

Competent MC1061 (F-) E. coli and nitrocellulose were purchased from Bio-Rad Laboratories. Pansorbin (Protein A-bearing S. aureus) cells were obtained from Calbiochem (San Diego, CA). K91 (F+) and MCI061 (F-) strains of E. coli were provided by Steve Cwirla (Affymax). MAb 3-E7 was purchased from Gramsch Laboratories (Schwabhausen, FRO). u-PA was obtained from Jack Henkin (Abbott Laboratories).

A polyvalent fd phage library that displayed random hexapeptide sequences and contained 2 X 10⁸ independent recombinants was prepared (Ding, C., et al., 1995; Smith, M.M., et al, 1995). Peptides were synthesized and purified as described (Madison, E. L., et al. (1995) J. Biol. Chem. 270,7558-7562.). Each member of this library displayed an N-terminal extension from phage coat protein III (pIII) containing a randomized region of six amino acids fused to pIII, followed by a six residue linker sequence (SSGGSG) and the epitopes for mAb 179 and mAb 3-E7. Because neither t-PA or u-PA digests the pIII sequence, the antibody epitopes, or the flexible linker sequence, the loss of antibody epitopes from the phage surface upon incubation with either enzyme requires cleavage of the randomized peptide insert. Incubation of the library with t-PA, followed by removal of phage retaining the antibody epitopes, therefore, accomplishes the enrichment of phage clones whose random hexamer sequence can be cleaved by t-PA.

The detailed construction of the phage vector fAFFI-tether C (fTC) and the random hexapeptide library fAFF-TC-LIB has been previously described (Smith, M.M., et al, 1995). Control substrate phage frC-PL, which contained the physiological target sequence for u-PA and t-PA, was constructed by hybridizing the single stranded oligonucleotides and and then ligating the annealed, double stranded products into the Xho I/Kpn I-cut vector frC. All constructs were first transformed into MC1061 by electroporation and then transferred into K91.

### Measurement of Enzyme Concentrations.

Concentrations of functional t-PA and u-PA were measured by active site titration with 4-methylumbelliferyl p-guanidinobenzoate (Jameson, G., et al., (1973) Biochem. J. 131, 107-117) using a Perkin-Elmer LS 50B Luminescence Fluorometer as previously described (Madison, E.L., et al., (1995) J. Biol. Chem. **270**: 7558-7562). In addition, the enzymes were titrated with a standard PAI-1 preparation that had been previously titrated against a trypsin primary standard. Total enzyme concentrations were measured by ELISA.

The procedure used in producing substrate substraction libraries is outlined in FIGURE 2. The initial phage library was subjected to three rounds of high stringency selection with t-PA to assure the preparation of an intermediate library that is highly enriched for phage that are very good substrates of t-PA. This intermediate library was then digested at low stringency with u-PA to remove phage that are moderate or good substrates for u-PA. Substrate subtraction was accomplished after the protease digestion of phage by adding Mab 3E-7 and immobilized protein A (Pansorbin cells, Calbiochem, San Diego, CA) to the reaction mixture and precipitating the ternary complexes which contain the undigested phage.

In contrast to earlier three rounds of selections, phage remaining in solution were then discarded, and the precipitate containing the ternary complexes is resuspended. Phage that were preferentially cleaved by t-PA were then identified by their release from the ternary complexes by digestion at high stringency with t-PA.

### Preparation and Sequencing of DNA from Phage Clones.

DNA samples were prepared from identified phage clones as previously described (Ding, C., et al., (1995)). Briefly, phage are precipitated from a 1 ml overnight culture by adding 200 µl of 20% polyethylene glycol in 2.5 M NaCl. The mixture was incubated on ice for 30 min., and the phage pellet was collected by microcentrifugation for 5 min. The phage were resuspended in 40 µl lysis buffer (10 mM Tris-HCL, pH 7.6, 0.1 mM EDTA, 0.5% Triton X-100) and heated at 80 C for 15 min. Single stranded DNA was purified by phenol extraction and ethanol precipitation and sequenced according to the method of Sanger.

The kinetic analysis of particular clones is summarized in Table 1. Tables 2 and 3 summarize the sequences of other additional clones. Table 2 shows the sequences of 37 t-PA - selective phage clones isolated and functionally verified containing 32 distinct substrate sequences. For comparison, the sequences of six u-PA selective clones are listed in Table 2.

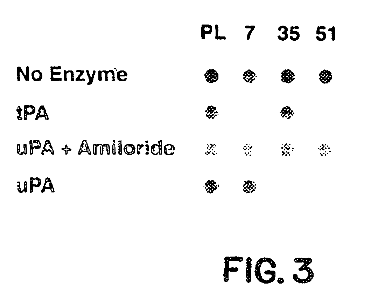

To verify that the substrate subtraction library had yielded substrates that were preferentially cleaved by t-PA, digestion of individual phage stocks by t-PA and u-PA was analyzed by a dot blot assay that was performed as previously described (Ding, L., et al., 1995; Smith, M.M., et al., 1995) (FIGURE 3). Loss of positive staining indicates loss of antibody epitopes from the phage due to proteolytic cleavage of the random hexamer region. Control phage PL contains the P3 - P3' region of the actual target sequence present in plasminogen (PGRVVG, residues 4-9 of SEQ ID NO:1) and was not digested by either enzyme under the conditions used in this test. Substrate phage 51 contained the hexamer RIARRA (SEQ ID NO:148) and was a substrate of both t-PA and u-PA. Phage 7 contained the hexamer FRGRAA (SEQ ID NO:25) and was a t-PA selective substrate. Phage 33 contained the hexamer RSANAI (SEQ ID NO:51) and was a u-PA selective substrate.

### Kinetics of Cleavage of Synthetic Peptides by t-PA and u-PA.

Individual phage stocks were prepared and digested with no enzyme, t-PA, u-PA, or u-PA in the presence of 1 mM amiloride, a specific inhibitor of u-PA. Kinetic data were obtained by incubating various concentrations of peptide with a constant enzyme concentration to achieve between 5 and 20% cleavage of the peptide in each reaction. For assays with u-PA, enzyme concentration was either 815 or 635 nM. For assays with t-PA enzyme concentration was 700 nM. Peptide concentrations were chosen where possible to surround Kₘ and in all cases were between 0.5 and 32 mM. The buffer used in these assays has been described (Madison, E. L., et al., 1995). Reactions were stopped by addition of triflouroacetic acid to 0.33 % or by freezing on dry ice. Cleavage of the 13 and 14 residue peptides was monitored by reverse phase HPLC as described (Madison, E. L., et al., 1995). The 4-6 residue peptides were acylated at their amino termini and amidated at their carboxyl termini. Cleavage of the 4-6 residue peptides was monitored by hydrophilic interaction HPLC chromatography (HILIC) (Alpert, A. J. (1990) J. Chromatog. 499, 177-196.) using a polyhydroxyaspartamine column from PolyLC (Columbia, MD). Buffer A was 50 mM triethylamine phosphate in 10% acetonitrile and buffer B was 10 mM triethylamine phosphate in 80% acetonitrile. Peptides were eluted by a gradient which was varied from 100% Buffer B to 100% Buffer A during a 13 minute interval. The percent of cleaved peptide was calculated by dividing the area under the product peaks by the total area under substrate and product peaks. For all peptides containing multiple basic residues, mass spectral analysis of products confirmed that cleavage occurred at a single site and identified the scissile bond. Data were interpreted by Eadie-Hofstee analysis. Errors were determined as described (Taylor, J. R., 1982) An introduction to error analysis. The study of uncertainties in physical measurements. University Science Books, Mill Valley, California.) and were <25%.

The results of kinetic analysis are summarized in Table 1, below, which compares the values determined for cleavage of the native target, plasminogen (I), t-PA selective peptides (II-X) and u-PA selective peptides (XI-XVIII).

Three peptide substrates (II - IV) containing hexamer sequences present in individual members of the substrate subtraction library were synthesized and characterized to provide a quantitative analysis of the properties of putative t-PA selective substrates. These peptides were cleaved between 13 and 47-fold more efficiently by t-PA than by u-PA (Table 1).

Comparison of the hexamer sequences obtained from the substrate subtraction library and the consensus sequences derived for substrates of u-PA and t-PA confirms the expected intimate similarity between optimal sub-site occupancy for these two closely related enzymes. In addition, these data strongly suggest that the P3 residue of a substrate is the primary determinant of the ability to distinguish between t-PA and u-PA. t-PA prefers arginine or large hydrophobic residues at this position while u-PA favors small hydrophilic residues, particularly serine.

In contrast to results obtained using t-PA, standard phage display was sufficient to yield highly selective u-PA substrates. One hundred substrate phage, containing 89 distinct random hexamer sequences, were selected using u-PA (Table 4). Dot blot analysis of the individual phage stocks under increasingly stringent conditions indicated that eleven clones, containing eight distinct hexamer sequences, were particularly labile u-PA substrates (Table 3). Peptides containing four of these eight hexamer sequences (XI - XIV) were synthesized and characterized. All four peptides were substantially improved substrates for u-PA, by factors of 840 - 5300, compared to a control peptide (I) that contained the actual target sequence present in plasminogen (Table 3). The four peptides were also cleaved 16 - 89 times more efficiently by u-PA than by t-PA.

To confirm the key role of P3 in defining specificity differences between t-PA and u-PA, variants of a u-PA selective peptide that contained either tyrosine or arginine at this position were synthesized and characterized. In striking contrast to parent peptide XI, the two variants (peptides VIII and IX) were cleaved 5.2-5.7 times more efficiently by t-PA than by u-PA, a 320-fold reversal in substrate preference (Table 1). Further replacement of the glycine found at P4 of the u-PA selective substrate with glutamine (peptide X) increased t-PA selectivity to 19-fold over u-PA. Point mutations at both P4 and P3, therefore, altered the relative specificity of t-PA versus u-PA by a factor of 1200.

The kinetic analysis described above was performed using substrate peptides that were 14 amino acids in length. To confirm that the specificity we observed was inherent in the selected hexapeptide sequences, and therefore would be expected to be readily converted into viable, small molecule peptidometics, we examined the kinetics of cleavage of short peptides containing only sequences found within selected hexapeptide sequences. For both t-PA and u-PA selective substrates, specificity was maintained by related pentapeptides. The peptide FRGRK was cleaved approximately 74 times more efficiently by t-PA than by u-PA while the peptide GSGKS was hydrolyzed approximately 120 times more efficiently by u-PA than by t-PA (Table 1). The relative specificity of these two pentapeptides for cleavage by t-PA versus u-PA, therefore, differs by a factor of approximately 9000, indicating that appropriate occupancy of the P4 - P1' subsites alone can mediate the ability of a substrate to distinguish the closely related enzymes t-PA and u-PA.

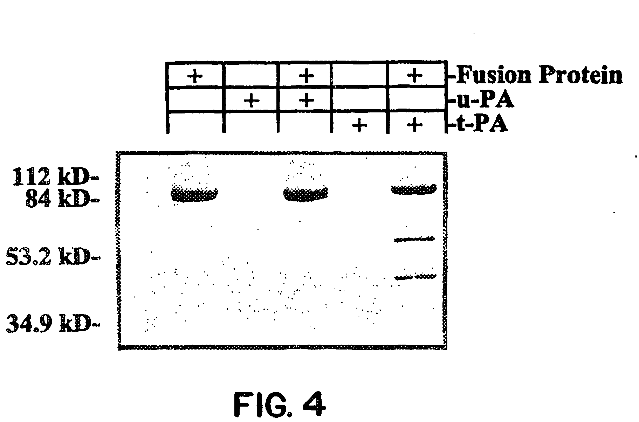

To define further the extent of substrate discrimination that could be achieved in other structural contexts, the t-PA selective hexapeptide QRGRSA was introduced into a fusion protein consisting of a photoreceptor protein linked to maltose binding protein. t-PA readily cleaved the fusion protein (FIGURE 4) whereas u-PA did not, demonstrating the maintenance of specificity for cleavage of this selected, primary sequence in the structural context of a protein substrate.

The fusion consists of maltose binding protein fused to the amino terminus of the HY4 gene product of Arabidopsis thaliana with a linking region in between coding for the amino acid sequence QRGRSA, which is cleaved by t-PA between R and S. The concentration of fusion protein in each lane is 1.4 mM and the concentration of u-PA or u-PA is 150 nM. The reaction buffer contains 50 mM Tris pH 7.5, 0.1 M NaCl, 1 mM EDTA, and 0.01 % Tween 20. Reactions were set up in a total volume of 20 mL and allowed to incubate 16 hours at 25 C and then stopped by the addition of 5 mL of 5X loading buffer and separated by electrophoresis on 12% polyacrylamide. The gel was stained with Coomassie Brilliant Blue 30 minutes and de-stained overnight.

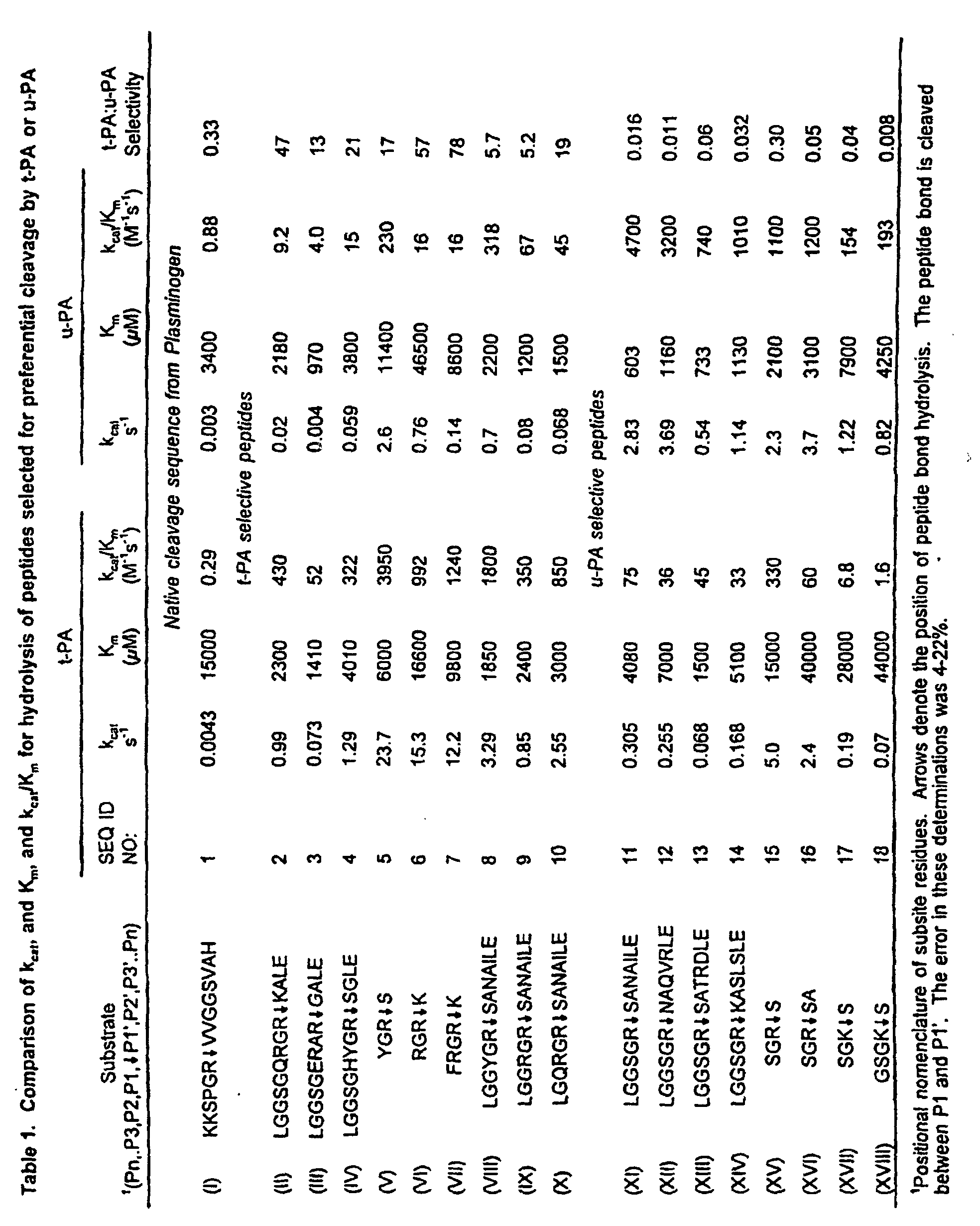

**Table 2:**

| **Primary sequences of hexamers of t-PA selective substrate clones** | | | | | | | | | | | |
|---|---|---|---|---|---|---|---|---|---|---|---|
| **SEQ ID** | **Clone** | **P5** | **P4** | **P3** | **P2** | **P1** | **P1'** | **P2'** | **P3'** | **P4'** | **P5'** |
| | | | | | | | | | | | |
| 19 | 1 | | | A | L | R | R | G | D | | |
| 20 | 2 | D | Y | R | G | R | M | (L) | | | |
| 21 | 3 | | E | R | A | R | G | A | | | |
| 22 | 4 | | E | R | L | R | K | A | | | |
| 23 | 5 | | | F | G | R | H | A | A | | |
| 24 | 6 | | F | L | | R | T | A | | | |
| 25 | 7 | | F | R | G | R | A | A | | | |
| 26 | 8 | | H | R | M | R | M | G | | | |
| 27 | 9 | | H | Y | G | R | S | G | | | |
| 28 | 10 | | | I | M | R | R | G | K | | |
| 29 | 11 | I | T | Y | G | R | R | (L) | | | |
| 30 | 12 | | K | F | T | R | S | G | | | |
| 31 | 13 | | L | I | P | R | R | A | | | |
| 32 | 14 | M | T | R | K | R | M | (L) | | | |
| 33 | 15 | | N | F | A | R | M | G | | | |
| 34 | 16 | | N | H | L | R | K | A | | | |
| 35 | 17 | | N | V | G | R | M | G | | | |
| 36 | 18 | | N | V | S | R | R | G | | | |
| 37 | 19 | | P | I | S | R | R | A | | | |
| 38 | 20 | | P | V | G | R | M | G | | | |
| 39 | 21 | | Q | R | G | R | K | A | | | |
| 40 | 22 | | R | L | L | R | S | V | | | |
| 41 | 23 | | S | F | G | R | R | H | | | |
| 42 | 24 | S | L | R | G | R | S | (L) | | | |
| 43 | 25 | | T | V | L | R | R | A | | | |
| 44 | 26 | | | V | A | R | R | V | K | | |
| 45 | 27 | | V | I | A | R | S | N | | | |
| 46 | 28 | | V | N | T | K | S | G | | | |
| 47 | 29 | | V | R | A | R | G | A | | | |
| 48 | 30 | V | R | R | G | R | S | (L) | | | |
| 49 | 31 | | V | R | R | R | G | A | | | |
| 50 | 32 | | T | R | V | R | A | K | | | |

**Table 3:**

| **Primary sequences of hexamers of most labile u-PA substrate clones** | | | | | | | | | | | |
|---|---|---|---|---|---|---|---|---|---|---|---|
| **SEQ ID** | **Clone** | **P5** | **P4** | **P3** | **P2** | **P1** | **P1'** | **P2'** | **P3'** | **P4'** | **P5'** |
| 51 | 33 | | | (S | G) | R | S | A | N | A | I |
| 52 | 34 | | | (S | G) | R | N | A | Q | V | R |
| 53 | 35 | | | (S | G) | R | S | A | T | R | D |
| 54 | 36 | | | (S | G) | R | S | A | K | V | D |
| 55 | 37 | | | (S | G) | R | K | A | S | L | S |
| 56 | 38 | | | (S | G) | R | R | A | V | S | N |
| 57 | 39 | | | (S | G) | R | S | A | V | V | K |
| 58 | 40 | | | (S | G) | R | S | S | S | S | H |

**TABLE 4:**

| **u-PA-Phage Selection Summary** | | | | | | | | | | | |
|---|---|---|---|---|---|---|---|---|---|---|---|
| **SEQ ID** | **SELECTIVITY** | **P5** | **P4** | **P3** | **P2** | **P1** | **P1'** | **P2'** | **P3'** | **P4'** | **P5'** |
| 59 | (U,T) | | A | I | K | R | S | A | | | |
| 60 | (U) | | | | G | R | R | G | N | R | |
| 61 | (U) | | | | G | R | S | V | N | N | |
| 62 | (U) | | | H | T | R | R | M | K | | |
| 63 | (U,T) | I | S | T | A | R | M | | | | |
| 64 | (U) | | | | | K | A | A | D | V | T |
| 65 | (U) | | | | | K | K | R | T | N | D |
| 66 | (U) | K | M | S | A | R | I | | | | |
| 67 | (U) | | | | K | R | R | D | V | A | |
| 68 | (U) | | | | K | R | V | S | K | N | |
| 69 | (U) | | | | | K | S | A | D | A | A |
| 70 | (U) | | | | | R | A | A | A | M | V |
| 71 | (U) | | | | | R | A | G | N | I | R |
| 72 | (U) | | | | | R | A | H | R | D | N |
| 73 | (U) | | | | | R | A | R | D | D | R |
| 74 | (U) | | | | | R | A | R | H | M | V |
| 75 | (U) | | | | | R | A | R | S | P | R |
| 76 | (U) | | | | | R | A | V | G | H | Q |
| 77 | (U) | | | | | R | A | V | V | D | S |
| 78 | (U) | | | | | R | G | G | K | G | P |
| 79 | (U,T) | | | | | R | G | R | S | A | V |
| 80 | (U) | | | | | R | G | V | D | M | N |
| 81 | (U) | | | | | R | G | V | K | M | H |
| 82 | (U) | | | | | R | H | R | S | D | I |
| 83 | (U) | | | | | R | K | G | Q | G | G |
| 84 | (U) | | | | | R | K | L | H | M | N |
| 85 | (U) | | | | | R | K | M | D | M | G |
| 86 | (U) | | | | | R | K | M | D | R | S |
| 87 | (U) | | | | | R | K | M | R | M | G |
| 88 | (U) | | | | | R | K | N | Q | R | V |
| 89 | (U) | | | | | R | K | Q | R | D | S |
| 90 | (U) | | | | | R | K | R | V | G | A |
| 91 | (U) | | | | | R | K | S | K | V | V |
| 92 | (U) | | | | | R | K | S | T | S | S |
| 93 | (U) | | | | | R | K | V | G | S | L |
| 94 | (U) | | | | | R | K | V | P | G | S |
| 95 | (U) | | | | | R | K | W | I | S | G |
| 96 | (U) | | | | | R | L | A | T | K | A |
| 97 | (U) | | | | | R | M | R | K | N | D |
| 98 | (U) | | | | | R | N | A | Q | V | R |
| 99 | (U,T) | | | | | R | N | A | V | E | P |
| 100 | (U) | | | | | R | N | D | R | L | N |
| 101 | (U) | | | | | R | N | G | K | S | R |
| 102 | (U) | | | | | R | N | M | P | L | L |
| 103 | (U) | | | | | R | N | T | G | S | H |
| 104 | (U) | | | | | R | R | M | T | M | G |
| 105 | (U) | | | | | R | R | R | L | N | M |
| 106 | (U) | | | | | R | R | T | L | D | F |
| 107 | (U,T) | | | | | R | S | A | K | V | D |
| 108 | (U) | | | | | R | S | A | N | A | I |
| 109 | (U) | | | | | R | S | A | T | R | D |
| 110 | (U) | | | | | R | S | A | V | V | K |
| 111 | (U) | | | | | R | S | D | Q | F | L |
| 112 | (U) | | | | | R | S | D | N | P | N |
| 113 | (U) | | | | | R | S | E | R | S | L |
| 114 | (U) | | | | | R | S | G | D | P | G |
| 115 | (U) | | | | | R | S | G | N | T | T |
| 116 | (U) | | | | | R | S | G | N | M | G |
| 117 | (U) | | | | | R | S | N | G | V | G |
| 118 | (U) | | | | | R | S | P | D | G | M |
| 119 | (U) | | | | | R | S | R | R | L | P |
| 120 | (U) | | | | | R | S | R | V | T | S |
| 121 | (U) | | | | | R | S | S | H | S | S |
| 122 | (U) | | | | | R | S | S | Q | A | A |
| 123 | (U) | | | | | R | S | S | S | S | H |
| 124 | (U) | | | | | R | S | S | S | T | V |
| 125 | (U) | | | | | R | S | T | D | L | G |
| 126 | (U) | | | | | R | S | T | N | V | E |
| 127 | (U) | | | | | R | S | T | R | H | K |
| 128 | (U) | | | | | R | S | Y | T | N | S |
| 129 | (U) | | | | | R | T | S | P | S | T |
| 130 | (U) | | | | | R | T | S | V | N | L |
| 131 | (U) | | | S | G | R | A | R | Q | | |
| 132 | (U) | | | S | K | R | A | S | I | | |
| 133 | (U) | | | | S | K | S | G | R | S | |
| 134 | (U) S | Q | T | C | V | R | | | | | |
| 135 | (U) | | | S | S | R | N | A | D | | |
| 136 | (U) | | | T | A | R | L | R | G | | |
| 137 | (U) | | | T | A | R | S | D | N | | |
| 138 | (U) | | | T | E | R | R | V | R | | |
| 139 | (U) | | | T | Q | R | S | T | G | | |
| 140 | (U) | | | | T | R | R | D | R | I | |
| 141 | (U) | | | T | S | R | M | G | T | | |
| 142 | (U) | | | T | S | R | Q | A | Q | | |
| 143 | (U) | | | T | T | R | R | N | K | | |
| 144 | (U) | | T | T | S | R | R | S | | | |
| 145 | (U,T) | | | V | A | R | M | Y | K | | |
| 146 | (U,T) | | | V | S | R | R | N | M | | |
| 147 | (U,T) | | W | S | G | R | S | G | | | |

### EXAMPLE 2:

### Preparation of Specific Inhibitors of t-PA

Specific inhibitors of t-PA were designed using the sequences derived in Example 1 by making variants of the PAI-1, natural inhibitor of t-PA.

### Site-directed Mutagenesis and Construction of an Expression Vector Encoding a Recombinant Variant of PAI-1.

The expression vector pPAIST7HS was derived from the plasmid pBR322 and contained a full length cDNA encoding human PAI-1 that was transcribed from a T7 gene 10 promoter (Tucker, H. M., et al., (1995) Nature Struct. Biol. **2**: 442-445). The 300 bp Sal I/Bam HI fragment of human PAI-1 was subcloned from pPAIST7HS into bacteriophage M13mp18. Single stranded DNA produced by the recombinant M13mp18 constructs was used as a template for site specific mutagenesis according to the method of Zoller and Smith (Zoller, M. I., and Smith, M. (1984) DNA 3, 479-488) as modified by Kunkel (Kunkel, T. A. (1985) Proc. Natl. Acad. Sci. U.S.A. 82, 488-492).

Expression of wild type and the mutated variant of PAI-1 was accomplished in the E. coli strain BL21 [DE3]pLys^{s} (Novagen, Madison, WI) which synthesizes T7 RNA polymerase in the presence of isopropylothio-B-D-galactoside (IPTG). Bacterial cultures were grown at 37 degrees Celsius with vigorous shaking to an A₅₉₅ of 0.9-1.1, and IPTG was added to a final concentration of 1 mM to induce the synthesis of T7 RNA polymerase and the production of PAI-1 proteins. Cultures were grown for an addition 1-2 hrs at 37 degrees Celsius and then shifted to 30 degrees Celsius for 2-6 hours.

Cells were pelleted by centrifugation at 8000 X g for 20 min at 4 degrees Celsius and resuspended in 40 ml of cold start buffer (20 mM Sodium Acetate, 200 mM NaCl and 0.01 % Tween 20, pH 5.6). The cell suspension was disrupted in a French pressure cell (Aminco), and cellular debris was removed by ultracentrifugation for 25 min at 32000 X g.

Purification of soluble, active PAI-1 was performed as previously described (Sancho, E., et al., (1994) Eur. J. Biochem. **224**: 125-134). PAI-1 containing supernatants were injected onto a XK-26 column (Pharmacia Biotech) packed with CM-50 Sephadex (Pharmacia). The column. was washed with 5 column volumes of start buffer (20 mM Sodium Acetate, 200 mM NaCl and 0.01% Tween 20, pH 5.6), and PAI-1 proteins were eluted using a 0.2 M - 1.8 M linear gradient of NaCl in the same buffer. Peak fractions were collected, pooled, and concentrated using a Centriplus 30 concentrator (Amicon). Purified preparations were analyzed by activity measurements using standard, direct assays of t-PA, SDS-PAGE, and measurement of optical density at 280 nm.

### Measurement of Active PAI-1 in Purified Preparations.

A primary standard of trypsin was prepared by active site titration using p-nitrophenyl-guanidinobenzoate HCl as described previously (Chase, **T.,** and Shaw, E. (1967) Biochem. Biophys. Res. Commun. **29:** 508-514). Concentrations of active molecules in purified preparations of wild type or mutated PAI-1's were determined by titration of standardised trypsin as described by Olson et al. (Olson, S. T., et al., (1995) J. Biol. Chem. **270:** 30007-30017) and by titration of standardized t-PA preparations.

### Kinetic Analysis of the Inhibition of t-PA and u-PA by Recombinant PAI-1 and PAI-1/UKI.

Second order rate constants (kᵢ) for inhibition of t-PA or u-PA were determined using pseudo-first order (kᵢ < 2 x 10⁶) or second order (kᵢ > 2 x 10⁶) conditions. For each reaction, the concentrations of enzyme and inhibitor were chosen to yield several data points for which the residual enzymatic activity varied between 20%-80% of the initial activity. Reaction conditions and data analysis for pseudo-first order reactions were as previously described.

For second order reactions, equimolar concentrations of u-PA and PAI-1 were mixed directly in microtiter plate wells and preincubated at room temperature for periods of time varying from 0 to 30 minutes. Following preincubation the mixtures were quenched with an excess of neutralizing anti-PAI-1 antibody (generously provided by Dr. David Loskutoff), and residual enzymatic activity was measured using a standard, indirect chromogenic assay. These indirect, chromogenic assays were compared to control reactions containing no PAI-1 or to which PAI-1 was added after preincubation, addition of anti-PAI-1 antibody, plasminogen, and Spec PL to the reaction mixture. Data were analyzed by plotting the reciprocal of the residual enzyme concentration versus the time of preincubation.

To test the prediction, based on an analysis of the cleavage of peptide substrates, that the P3 residues can mediate the ability of an inhibitor to discriminate between t-PA and u-PA, site-specific mutagenesis was performed on PAI-1, the primary physiological inhibitor of both t-PA and u-PA.

Three variants of PAI-1 were produced and characterized. The first was a variant in which the P3 serine (Ser 344) was converted to an arginine residue. The second was a variant in which the P4 valine (Val 343) was replaced by a glutamine residue. The third variant was a double mutant in which both of the substitutions were made.

Kinetic analysis of the inhibition of both t-PA and u-PA by these variants of PAI-1 was consistent with the tests based on the peptide substrates. The second-order rate constants for inhibition of t-PA and u-PA by wild type PAI-1 were 1.6 x 10⁶ M⁻¹s⁻¹ and 1.9 x 10⁷ M⁻¹s⁻¹, respectively. Thus, wild-type PAI-1 shows about 11.9-fold specificity toward u-PA.

In contrast, the second-order rate constants for inhibition of t-PA and u-PA by the P3 arginine mutant PAI-1 were, respectively, 1.4 x 10⁶ M⁻¹s⁻¹ and 1.0 x 10⁵ M⁻¹s⁻¹, an approximately 170-fold reversal in specificity toward t-PA. This large change in specificity was achieved without sacrificing activity toward the target enzyme. The P3 arginine mutation reduced activity of PAI-1 toward u-PA by a factor of about 190 without significantly affecting reactivity toward t-PA.

An individual mutation of the P4 valine to a glutamine had no effect on the rate of inhibition of either t-PA or u-PA. As suggested by the predominance in the subtraction library of substrates containing both large P3 and large P4 residues, the P4 glutamine mutation did increase the t-PA selectivity of the P3 arginine variant of PAI-1. The second order rate constants for the inhibition of t-PA and u-PA by this double mutant PAI-1 were, respectively, 1.4 x 10⁶ M⁻¹s⁻¹ and 2.9 x 10⁴ M⁻¹s⁻¹. While maintaining full activity toward t-PA, the double mutant showed an approximately 600-fold enhanced t-PA/u-PA selectivity compared to wild-type PAI-1 and about a 3.5-fold greater t-PA selectivity than the P3 arginine variant of PAI-1. The absolute t-PA/u-PA selectivity of wild-type PAI-1, the P3 arginine single mutant and the P3 Arg, P4 Gln double mutant was 0.08, 14, and 48, respectively.

**Table 5:**

| **Second order rate constants for inhibition of t-PA or u-PA by wild type PAI-1 and variants of PAI-1** | | | | | |
|---|---|---|---|---|---|
| Inhibitor | SEQ ID NO: | Primary Sequence of reactive center loop (P4-P2') | Rate constant toward t-PA M⁻¹s⁻¹ | Rate constant toward u-PA M⁻¹s⁻¹ | t-PA/u-PA Selectivity |
| Wild type PAI-1 | 149 | VSAR↓MA | 1.6 x 10⁶ | 1.9 x 10⁷ | 0.08 |
| PAI-1/P3R | 150 | VRAR↓MA | 1.4 x 10⁶ | 1.0 x 10⁵ | 14 |
| PAI-1/P4Q | 151 | QSAR↓MA | 1.6 x 10⁶ | 1.9 x 10⁷ | 0.08 |
| PAI-1/P4Q,P3R | 152 | QRAR↓MA | 1.4 x 10⁶ | 2.9 x 10⁴ | 48 |

### EXAMPLE 3:

### Preparation of Specific Inhibitors of Urokinase

Substrate phage display alone, without subtractive substrate screening, was to identify peptides that are cleaved 840-5300 times more efficiently by u-PA than peptides containing the wild type physiological target sequence of the enzyme. In addition, the peptide substrates selected were cleaved as much as 120 times more efficiently by u-PA than by t-PA.

In general, with the exception of the screening protocol, procedures followed were those used in Example 1. Digestion of the phage was performed using enzyme concentrations varying from 2 - 10 µg/ml and incubation times varying from 0.5 - 10 hours. Phage precipitation and dot blot analysis were performed as described in Example 1. Individual phage stocks were prepared and digested with no enzyme, t-PA, u-PA, or u-PA in the presence of 1 mM amiloride, a specific inhibitor of u-PA' for periods of time varying from 15 minutes to 10 hours. Individual reaction mixtures were spotted onto a nitrocellulose filter using a dot blotter apparatus (BioRad). The filter was probed with MAb 3E-7 and developed using the Amersham Western ECL kit. Loss of positive staining indicates loss of antibody epitopes from the phage due to proteolytic cleavage of the randomized hexamer region.

Kinetic data were obtained by incubating various concentrations of peptide with a constant enzyme concentration to achieve between 5 and 20% cleavage of the peptide in each reaction as described in Example 1.

A polyvalent fd phage library that displayed random hexapeptide sequences and contained 2 X 10⁸ independent recombinants was prepared. Each member of this library displayed an N-terminal extension from phage coat protein III (pIII) that contained a randomized region of six amino acids, a six residue linker sequence (SSGGSG), and the epitopes for mAb 179 and mAb 3-E7. Because u-PA did not digest the pIII sequence, the antibody epitopes, or the flexible linker sequence, the loss of antibody epitopes from the phage surface upon incubation with u-PA required cleavage of the randomized peptide insert. Incubation of the library with u-PA, followed by removal of phage retaining the antibody epitopes, therefore, accomplished a large enrichment of phage clones whose random hexamer sequence could be cleaved by u-PA.

Following five rounds of selection to enrich and amplify phage which display sequences that are readily cleaved by u-PA, 100 phage clones were identified as u-PA substrates. DNA sequencing of these clones revealed the presence of 91 distinct hexamer sequences among the selected phage (Table 6, below). As expected from the trypsin-like primary specificity of u-PA, each hexamer contained at least one basic residue, and 89 of the 91 hexamer sequences contained at least one arginine residue. 35 of the 91 substrate phage contained a single basic residue, and in 33 of these 35 cases the single basic residue was an arginine. An additional 22 phage contained two basic residues but only a single arginine. Alignment and analysis of these hexamer sequences suggested that the consensus sequence for optimal subsite occupancy for substrates of u-PA, from P3 - P2', was SGR(S > R,K,A)X, where "X" represents a variety of amino acid residues but is most often alanine, glycine, serine, valine, or arginine.

Analysis of these data was complicated by the fact that approximately 72 % of the selected substrate phage contained an arginine in the first position of the randomized hexamer and therefore utilized the amino terminal flanking residues, Ser-Gly, to occupy the P3 and P2 subsites. While these results left no doubt that the P3-PT SGR sequence created by the fusion was a very favorable recognition site for u-PA, this use of flanking residues necessitated a particularly careful examination of the P3 and P2 preferences of u-PA.

Two changes were made in the experimental protocol to examine the P3 and P2 preferences of u-PA. First, an unusually large collection of substrate phage (91) were isolated to assure that a reasonable number of these (23) would not utilize the flanking Ser-Gly to fill the P3 and P2 subsites. This allowed a meaningful comparison of the consensus sequence derived from the entire library with that derived from the non-fusion phage and the demonstration of good agreement between the two consensus sequences. Second, dot blot analysis was performed as described in Example 1 on all 100 substrate phage using a wide variety of stringencies of digestion by u-PA. Although this semi-quantitative assay cannot provide kinetic constants, it can provide an accurate rank ordering of the lability of the substrate phage clones.

Under the most stringent conditions examined, 11 of the 100 substrate phage, containing 8 distinct randomized hexamer sequences, proved to be particularly labile u-PA substrates. The sequences were the same as those listed in Table 3, above. All 8 of the most labile substrate phage contained the P3-P1 SGR motif, demonstrating that this sequence is, in fact, a more labile u-PA site than related, selected sequences present in the library such as SSR, TAR, TSR, TTR, etc. This dot blot analysis also yielded additional information regarding the preferences of u-PA for the unprimed subsites. While analysis of the entire substrate phage library failed to reveal a clear consensus at P1' and P2' the most labile substrate phage displayed an obvious preference at both of these positions. Five of the eight most labile phage contained a serine residue at P1', and seven of these eight phage contained an alanine residue at P2'. These observations strongly suggest that the primary sequence SGRSA, from P3-P2', represents optimal subsite occupancy for substrates of u-PA.

**Table 6.**

| **Amino acid sequences of the hexapeptide in 89 isotated substrate phage clones.** | | |
|---|---|---|
| **Clone Number** | **Amino acid Sequence** | **SEQ ID NO**: |
| 1 | S G R A R Q | 153 |
| 2 | S K S G R S(L) | 154 |
| 3 | S S R N A D | 155 |
| 4 | T A R L R G | 156 |
| 5 | T A R S D N | 157 |
| 6 | T S R M G T | 158 |
| 7 | T S R Q A Q | 159 |
| 8 | T T R R N K | 160 |
| 9 | T T S R R S | 161 |
| 10 | W S G R S G | 162 |
| 11 | A I K R S A | 163 |
| 12 | (G)G R R G N R | 164 |
| 13 | (G)G R S V N N | 165 |
| 14 | H T R R M K | 166 |
| 15 | I S T A R M(L) | 167 |
| 16 | (S G)K A A D V T | 168 |
| 17 | K K R T N D | 169 |
| 18 | K M S A R I(L) | 170 |
| 19 | (G)K R R D V A | 171 |
| 20 | (G)K R V S K N | 172 |
| 21 | (S G)K S A D A A | 173 |
| 22 | (S G)R A A A M | 174 |
| 23 | (S G)R A G N I R | 175 |
| 24 | (S G)R A H R D N | 176 |
| 25 | (S G)R A R D D R | 177 |
| 26 | (S G)R A R H M | 178 |
| 27 | (S G)R A R S P R | 179 |
| 28 | (S G)R A V G H Q | 180 |
| 29 | (S G)R A V V D S | 181 |
| 30 | (S G)R G G K G P | 182 |
| 31 | (S G)R G R S A V | 183 |
| 32 | (S G)R G V D M N | 184 |
| 33 | (S G)R G V K M H | 185 |
| 34 | (S G)R H R S D I | 186 |
| 35 | (S G)R K G Q G G | 187 |
| 36 | (S G)R K L H M N | 188 |
| 37 | (S G)R K M D M G | 189 |
| 38 | (S G)R K M D R S | 190 |
| 39 | (S G)R K M R M G | 191 |
| 40 | (S G)R K N Q R V | 192 |
| 41 | (S G)R K Q R D S | 193 |
| 42 | (S G)R K R V G A | 194 |
| 43 | (S G)R K S K V V | 195 |
| 44 | (S G)R K S T S S | 196 |
| 45 | (S G)R K V G S L | 197 |
| 46 | ( S G)R K A S L S | 37 |
| 47 | (S G)R K V P G S | 198 |
| 48 | (S G)R K W I S G | 199 |
| 49 | (S G)R L A T K A | 200 |
| 50 | (S G)R M R K N D | 201 |
| 51 | (S G)R N A Q V R | 34 |
| 52 | (S G)R N A V E P | 202 |
| 53 | (S G)R N D R L N | 203 |
| 54 | (S G)R N G K S R | 204 |
| 55 | (S G)R N M P L L | 205 |
| 56 | (S G)R N T G S H | 206 |
| 57 | (S G)R R M T M G | 207 |
| 58 | (S G)R R R L N M | 208 |
| 59 | (S G)R R T L D F | 209 |
| 60 | (S G)R R A V S N | 38 |
| 61 | (S G)R S A K V D | 36 |
| 62 | (S G)R S A N A I | 33 |
| 63 | (S G)R S A T R D | 35 |
| 64 | (S G)R S A V V K | 39 |
| 65 | (S G)R S D Q F L | 210 |
| 66 | (S G)R S D N P N | 211 |
| 67 | (S G)R S E R S L | 212 |
| 68 | (S G)R S G D P G | 213 |
| 69 | (S G)R S G N T T | 214 |
| 70 | (S G)R S G N M G | 215 |
| 71 | (S G)R S N G V G | 216 |
| 72 | (S G)R S P D G M | 217 |
| 73 | (S G)R S R R L P | 218 |
| 74 | (S G)R S R V T S | 219 |
| 75 | (S G)R S S H S S | 220 |
| 76 | (S G)R S S Q A A | 221 |
| 77 | (S G)R S S S S H | 40 |
| 78 | (S G)R S S S T V | 222 |
| 79 | (S G)R S T D L G | 223 |
| 80 | (S G)R S T N V E | 224 |
| 81 | (S G)R S T R H K | 225 |
| 82 | (S G)R S Y T N S | 226 |
| 83 | (S G)R T S P S T | 227 |
| 84 | (S G)R T S V N L | 228 |
| 85 | S K R A S I | 229 |
| 86 | S Q T C V R(L V) | 230 |
| 87 | T E R R V R(L V) | 231 |
| 88 | T Q R S T G | 232 |
| 89 | T R R D R I | 233 |
| 90 | V A R M Y K | 234 |
| 91 | V S R R N M | 235 |

### Kinetic analysis of the cleavage of neptides containing sequences present in selected substrate phage.

Four peptides containing amino acid sequences present in the randomized hexamer region of the most labile phage were chosen for detailed kinetic analysis (Table 7) and compared to hydrolysis of a control peptide (I) containing the P3-P4' sequence of plasminogen, a series of residues which fall within a disulfide-linked loop in the native protein. All four of the selected peptides were substantially improved substrates for u-PA, by factors of 840 - 5300, compared with the control, plasminogen peptide (Table 7). These increases in catalytic efficiency were mediated primarily by increases in k_{cat}, suggesting that optimized subsite interactions served to lower the energy of the transition state rather than the ground state. For example, compared with that of control peptide (I), the Kₘ for cleavage of the most labile, selected peptide (II) was reduced by a factor of 5.6. However, the k_{cat} was increased by a factor of more than 940. In addition, peptide substrates that interacted optimally with the primary subsites of u-PA were selective for cleavage by u-PA relative to t-PA. The four selected peptides (II - V), for example, were cleaved 16-89 times more efficiently by u-PA than by t-PA, and improvements in both Kₘ and k_{cat} contributed to the preferential hydrolysis by u-PA.

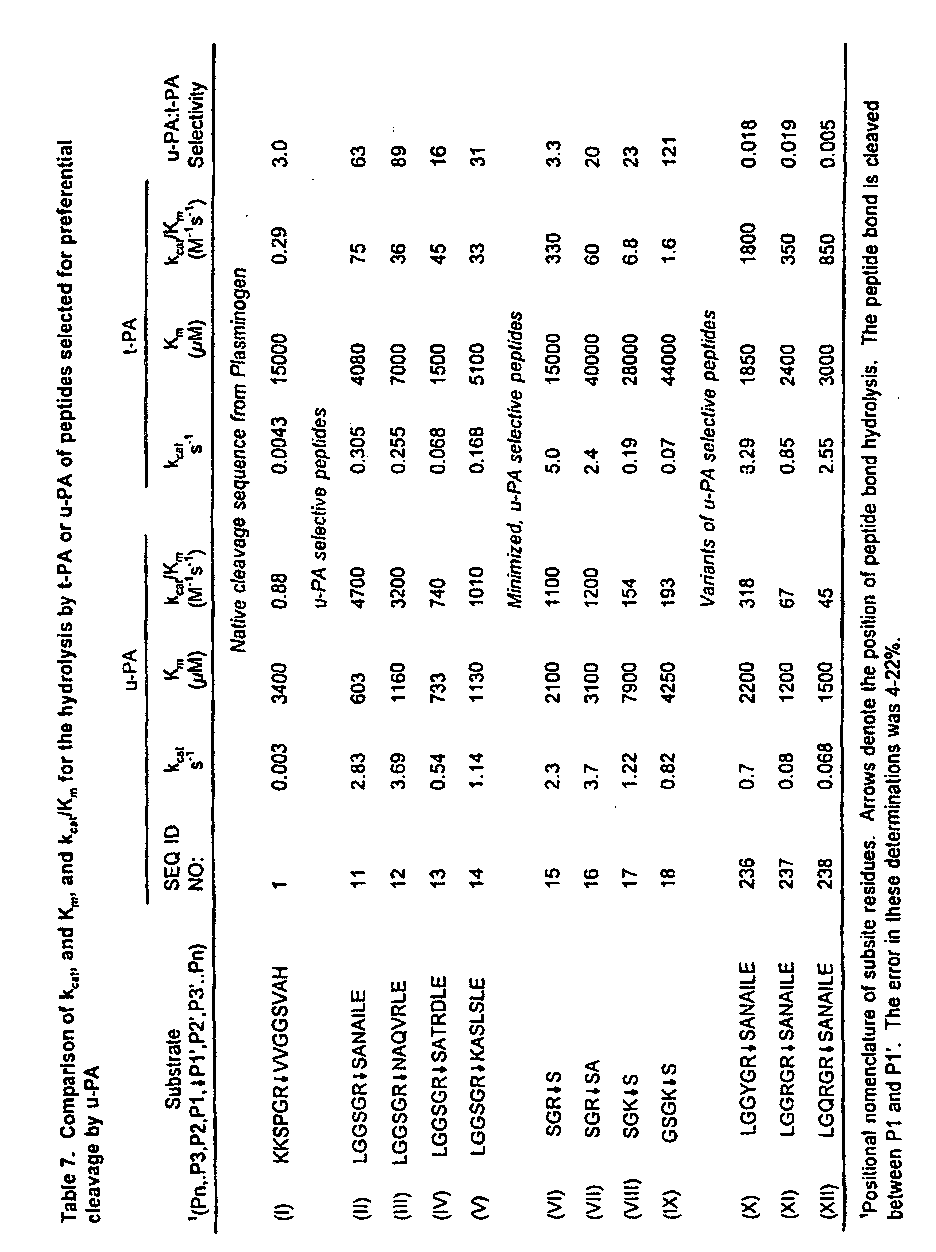

### Minimization of the Selective Peptide Substrates,

The kinetic analysis described above was performed using substrate peptides that were 14 amino acids in length. To confirm that the specificity observed was inherent in the selected hexapeptide sequences, the kinetics of cleavage of short peptides containing only sequences found within selected hexapeptide sequences was examined. Pentapeptide VII, for example, was cleaved by u-PA with a catalytic efficiency of 1200 M⁻¹s⁻¹ and exhibited a u-PA/t-PA selectivity of 20. The behavior of pentamer VII in these assays, therefore, was very similar to that of peptide IV, a 14-mer that contains the same P3-P2' sequence as the pentamer. These observations indicate that appropriate occupancy of the P3-P2' subsites alone can create selective substrates for u-PA.

Differences at position 190 (chymotrypsin numbering system) between u-PA and t-PA suggest that u-PA may exhibit decreased discrimination between arginine and lysine at the P1 position of a substrate compared with t-PA. Consistent with this hypothesis and by contrast to the selected t-PA substrate library, the u-PA library did include members that contained a P1 lysine. This observation suggested that the u-PA/t-PA selectivity of a peptide substrate should be enhanced by placement of lysine in the P1 position although this increased selectivity was likely to be accompanied by decreased reactivity toward u-PA. To test this hypothesis we analyzed hydrolysis of a variant of u-PA selective peptide (VI) that contained a P1 lysine (peptide VIII). The P1 lysine mutation decreased the catalytic efficiency for cleavage of this peptide by a factor of 49 for t-PA and by a factor of 7 for u-PA. As predicted, then, the P1 lysine mutation did enhance the u-PA/t-PA selectivity of the peptide substrate by a factor of approximately 7. It is not surprising, therefore, that the most selective u-PA substrate, peptide IX which is cleaved approximately 121 times more efficiently by u-PA than by t-PA, is derived from the randomized hexamer region of a substrate phage that contained a P1 lysine.

### Importance of P3 and P4 for discrimination between u-PA and t-PA.

Recent investigations that explored optimal subsite occupancy for substrates of t-PA suggested that the P3 residue was the primary determinant of the ability of a substrate to discriminate between t-PA and u-PA and that this selectivity could be enhanced modestly by appropriate occupancy of P4. These suggestions were based on evidence obtained from a statistical analysis of phage selected using a substrate subtraction protocol rather than by a kinetic analysis of peptide substrates. Consequently, to test these hypotheses, we synthesized variants of the most labile u-PA selective substrate (peptide II) that contained mutations in the P3 and/or P4 positions and analyzed the hydrolysis of these peptides by u-PA and t-PA. In peptide X the P3 Serine of peptide II was replaced by a tyrosine, and in peptide XI the P3 serine was replaced by arginine. As expected, these mutations substantially decreased the u-PA/t-PA selectivity of the peptide by factors of 330 or 360, respectively, and actually converted the peptide into a t-PA selective substrate. Moreover, mutation of both the P3 serine and P4 glycine of the most labile u-PA substrate to arginine and glutamine, respectively (peptide XII), decreased the u-PA/t-PA selectivity by a factor of 1200. These data confirm the proposed status of the P3 and P4 residues as specificity determinants for substrates of t-PA and u-PA and suggest a particularly prominent role of the P3 residue in this capacity.

### EXAMPLE 4:

### Design and Characterization of a Variant of PAI-1 That is Selective for u-PA.

Analysis of the selected peptide substrates identified in Example 3 indicated that the primary sequence SGRSA, from positions P3 to P2', represented an optimal subsite occupancy for substrates of u-PA. This information was to design a variant of plasminogen activator inhibitor type 1 (PAI-1), the primary physiological inhibitor of both u-PA and t-PA, that inhibited u-PA approximately 70 times more rapidly than it inhibited t-PA.

Specific inhibitors of u-PA were designed using the procedure described in Example 2 by making variants of the PAI-1. Oligonucleotide directed, site specific mutagenesis was used as described in Example 2 to construct a variant of PAI-1 that contained the primary sequence found in the peptide substrate that was most selective for u-PA, GSGKS, from the P4 - P1' position of the reactive center loop. The mutagenic oligonucleotide had the sequence

Following mutagenesis, single-stranded DNA corresponding to the entire 300-bp SalI-BamHI fragment was fully sequenced to ensure the presence of the desired mutations and the absence of any additional mutations The 300-bp SalI-BamHI dsDNA fragment from the mutated, replicative form DNA was used to replace the corresponding fragment in pPAIST7HS to yield a full-length cDNA encoding PAI-1/UK1, which contained the amino acid sequence GSGKSA from the P4 to P2' positions of the reactive center loop.

Kinetic analysis indicated that the PAI-1 variant inhibited u-PA approximately 70 times more rapidly than it inhibited t-PA with second order rate constants for inhibition of u-PA and t-PA of 6.2 X 10⁶ M⁻¹s⁻¹ and 9 X 10⁴ M⁻¹s⁻¹, respectively. By contrast, wild type PAI-1 inhibits u-PA and t-PA with second order rate constants of 1.9 X 10⁷ M⁻¹s⁻¹ Z and 1.8 X 10⁶ M⁻¹s⁻¹ respectively. As anticipated, therefore, the mutated serpin possessed a u-PA/t-PA selectivity that was approximately 7-fold greater than that of wild type PAI-1. Moreover, the 70-fold selectivity of the PAI-I variant is consistent with the value of 120 observed for hydrolysis of the corresponding peptide substrate by the two enzymes (Tables 7 and 8).

**Table 8:**

| **Second order rate constants for inhibition of t-PA or u-PA by wild type PAI-1 and variant PAI-1/UK1** | | | | | |
|---|---|---|---|---|---|
| Inhibitor | SEQ ID NO: | Primary Sequence of reactive center loop (P4-P2') | Rate constant toward u-PA M⁻¹s⁻¹ | Rate constant toward t-PA M⁻¹s⁻¹ | t-PA/u-PA Selectivity |
| Wild type PAI-1 | 149 | VSAR↓MA | 1.9 × 10⁷ | 1.8 × 10⁶ | 11 |
| PAI-1/UK1 | 239 | GSGK↓SA | 6.2 × 10⁶ | 9.0 × 10⁴ | 69 |

Other embodiments of the present invention will be apparent to those skilled in the arts of protein engineering or rational drug design.

## Claims

1. A method of producing a substrate subtraction library of peptides wherein each peptide is a substrate for a first peptide-modifying enzyme but is not a substrate for a second peptide-modifying enzyme, comprising the steps of:
a) providing a combinatorial library comprising components that display different peptides;
b) contacting the combinatorial library with a first peptide-modifying enzyme to permit the first enzyme to modify peptides in the library to form a first modified peptide component portion and a first unmodified peptide component portion of the library;
c) separating the first modified portion of the library from the first unmodified portion of the library;
d) contacting the first modified portion of the library with a second peptide-modifying enzyme to permit the second enzyme to modify peptides in the first modified portion of the library to form a second modified peptide component portion and a second unmodified peptide component portion of the library;
e) separating the second modified portion of the library from the second unmodified portion of the library; and
f) retaining the second unmodified portion to form a library of peptides that are each a substrate for the first enzyme but not a substrate for the second enzyme.

2. The method of claim 1, wherein the first enzyme and the second enzyme are proteases.

3. The method of claim 1, wherein the first enzyme comprises tissue plasminogen activator (t-PA) and the second enzyme comprises urokinase-type plasminogen activator (u-PA).

4. The method of claim 1, wherein the first enzyme comprises urokinase-type plasminogen activator (u-PA) and the second enzyme comprises tissue plasminogen activator (t-PA).

5. The method of claim 1, wherein the first enzyme and the second enzyme are kinases.

6. The method of claim 1, wherein the first enzyme and the second enzyme are phosphatases.

7. The method of claim 1, wherein the combinatorial library is a bacteriophage display library.

8. A method for producing a substrate subtraction library of peptides wherein each peptide is a substrate for a second peptide-modifying enzyme but is not a substrate for a first peptide-modifying enzyme, comprising the steps of:
a) providing a combinatorial library comprising components that display different peptides;
b) contacting the combinatorial library with a first peptide-modifying enzyme to permit the first enzyme to modify peptides in the library to form a first modified peptide component portion and a first unmodified peptide component portion of the library;
c) separating the first modified portion of the library from the first unmodified portion of the library;
d) contacting the first unmodified portion of the library with a second peptide-modifying enzyme to permit the second enzyme to modify peptides to form a second modified peptide component portion and a second unmodified peptide component portion of the library; and
e) separating the second modified portion of the library from the second unmodified portion of the library, and retaining the second modified portion to form a library of peptides that are each a substrate for the second enzyme but not a substrate for the first enzyme.

9. The method of claim 8, wherein the first enzyme and the second enzyme are proteases.

10. The method of claim 8, wherein the first enzyme comprises tissue plasminogen activator (t-PA) and the second enzyme comprises urokinase-type plasminogen activator (u-PA).

11. The method of claim 8, wherein the first enzyme comprises urokinase-type plasminogen activator (u-PA) and the second enzyme comprises tissue plasminogen activator (t-PA).

12. The method of claim 8, wherein the first enzyme and the second enzyme are kinases.

13. The method of claim 8, wherein the first enzyme and the second enzyme are phosphatases.

14. The method of claim 8, wherein the combinatorial library is a bacteriophage display library.

15. An isolated polypeptide, the amino acid sequence of which is selected from the group consisting of SEQ ID NO:2 to SEQ ID NO:5, SEQ ID NO:7 to SEQ ID NO:148 and SEQ ID NO:150 to SEQ ID NO:239.

16. The polypeptide of claim 15, wherein the amino acid sequence is selected from the group consisting of SEQ ID NO:2 to SEQ ID NO:5 and SEQ ID NO:7 to SEQ ID NO:30.

17. The polypeptide of claim 15, wherein the amino acid sequence is selected from the group consisting of SEQ ID NO:141 to SEQ ID NO:148.

18. The polypeptide of claim 15, wherein the amino acid sequence is selected from the group consisting of SEQ ID NO:150 to SEQ ID NO:239.

19. The polypeptide of claim 15, wherein the amino acid sequence is selected from the group consisting of SEQ ID NO:5 and SEQ ID NO:7 to SEQ ID NO:18.

20. The polypeptide of claim 15, wherein the amino acid sequence is SEQ ID NO: 150.

21. The polypeptide of claim 15, wherein the amino acid sequence is SEQ ID NO: 151.

22. The polypeptide of claim 15, wherein the amino acid sequence is SEQ ID NO:152.

23. The polypeptide of claim 15, wherein the amino acid sequence is SEQ ID NO:239.

## Patentansprüche

1. A Verfahren zur Herstellung einer Substrat-Subtraktions-Peptidbibliothek, worin jedes Peptid ein Substrat für ein erstes Peptid-modifizierendes Enzym ist, nicht aber ein Substrat für ein zweites Peptid-modifizierendes Enzym, das die folgenden Schritte umfasst:
a) das zur Verfügung stellen einer kombinatorischen Bibliothek, die Komponenten umfasst, die verschiedene Peptide zur Verfügung stellen;
b) das in Kontakt bringen der kombinatorischen Bibliothek mit einem ersten Peptid-modifizierenden Enzym, um es dem ersten Enzym zu ermöglichen, Peptide in der Bibliothek zu modifizieren, um einen ersten modifizierten Peptidkomponentenanteil und einen ersten nicht-modifizierten Peptidkomponentenanteil der Bibliothek auszubilden;
c) das Abtrennen des ersten modifizierten Anteils der Bibliothek von dem ersten nicht-modifizierten Anteil der Bibliothek;
d) das in Kontakt bringen des ersten modifizierten Anteils der Bibliothek mit einem zweiten Peptid-modifizierenden Enzym, um es dem zweiten Enzym zu ermöglichen, Peptide in dem ersten modifizierten Anteil der Bibliothek zu modifizieren, um einen zweiten modifizierten Peptidkomponentenanteil und einen zweiten nicht-modifizierten Peptidkomponentenanteil der Bibliothek auszubilden;
e) das Abtrennen des zweiten modifizierten Anteils der Bibliothek von dem zweiten nicht-modifizierten Anteil der Bibliothek; und
f) das Zurückbehalten des zweiten nicht-modifizierten Anteils, um eine Bibliothek von Peptiden auszubilden, die jeweils ein Substrat für das erste Enzym sind, nicht jedoch ein Substrat für das zweite Enzym sind.

2. Das Verfahren von Anspruch 1, worin das erste Enzym und das zweite Enzym Proteasen sind.

3. Das Verfahren von Anspruch 1, worin das erste Enzym den Gewebeplasminogenaktivator (t-PA) umfasst und das zweite Enzym den Urokinase-Typ Plasminogenaktivator (u-PA) umfasst.

4. Das Verfahren von Anspruch 1, worin das erste Enzym den Urokinase-Typ Plasminogenaktivator (u-PA) und das zweite Enzym den Gewebeplasminogenaktivator (t-PA) umfasst.

5. Das Verfahren von Anspruch 1, worin das erste Enzym und das zweite Enzym Kinasen sind.

6. Das Verfahren von Anspruch 1, worin das erste Enzym und das zweite Enzym Phosphatasen sind.

7. Das Verfahren von Anspruch 1, worin die kombinatorische Bibliothek eine Bakteriophagen-Display-Bibliothek ist.

8. Ein Verfahren zur Herstellung einer Substrat-Subtraktionspeptidbibliothek, worin jedes Peptid ein Substrat für ein zweites Peptid-modifizierendes Enzym ist, nicht aber ein Substrat für ein erstes Peptid-modifiziertes Enzym, das die folgenden Schritte umfasst:
a) das zur Verfügung stellen einer kombinatorischen Bibliothek, die Komponenten umfasst, die verschiedene Peptide zur Verfügung stellen;
b) das in Kontakt bringen der kombinatorischen Bibliothek mit einem ersten Peptid-modifizierenden Enzym, um es dem ersten Enzym zu ermöglichen, Peptide in der Bibliothek zu modifizieren, um einen ersten modifizierten Peptidkomponentenanteil und einen ersten nicht-modifizierten Peptidkomponentenanteil der Bibliothek auszubilden;
c) das Abtrennen des ersten modifizierten Anteils der Bibliothek von dem ersten nicht-modifizierten Anteil der Bibliothek;
d) das in Kontakt bringen des ersten nicht-modifizierten Anteils der Bibliothek mit einem zweiten Peptid-modifizierenden Enzym, um es dem zweiten Enzym zu ermöglichen, Peptide zu modifizieren, um einen zweiten modifizierten Peptidkomponentenanteil und einen zweiten nicht-modifizierten Peptidkomponentenanteil der Bibliothek auszubilden;
e) das Abtrennen des zweiten modifizierten Anteils der Bibliothek von dem zweiten nicht-modifizierten Anteil der Bibliothek und das Zurückbehalten des zweiten modifizierten Anteils, um eine Bibliothek von Peptiden auszubilden, die jeweils ein Substrat für das zweite Enzym, nicht aber ein Substrat für das erste Enzym sind.

9. Das Verfahren von Anspruch 8, worin das erste Enzym und das zweite Enzym Proteasen sind.

10. Das Verfahren von Anspruch 8, worin das erste Enzym den Gewebeplasminogenaktivator (t-PA) umfasst, und das zweite Enzym den Urokinase-Typ Plasminogenaktivator (u-PA) umfasst.

11. Das Verfahren von Anspruch 8, worin das erste Enzym den Urokinase-Typ Plasminogenaktivator (u-PA) umfasst und das zweite Enzym den Gewebeplasminogenaktivator (t-PA) umfasst.

12. Das Verfahren von Anspruch 8, worin das erste Enzym und das zweite Enzym Kinasen sind.

13. Das Verfahren von Anspruch 8, worin das erste Enzym und das zweite Enzym Phosphatasen sind.

14. Das Verfahren von Anspruch 8, worin die kombinatorische Bibliothek eine Bakteriaphagen-Display-Bibfiothek ist.

15. Ein isoliertes Polypeptid, dessen Aminosäuresequenz aus der Gruppe ausgewählt ist, die aus SEQ ID NO: 2 bis SEQ ID NO: 5, SEQ ID NO: 7 bis SEQ ID NO: 148 und SEQ ID NO: 150 bis SEQ ID NO: 239 besteht.

16. Das Polypeptid von Anspruch 15, worin die Aminosäuresequenz aus der Gruppe ausgewählt ist, die aus SEQ ID NO: 2 bis SEQ ID NO: 5 und SEQ ID NO: 7 bis SEQ ID NO: 30 besteht.

17. Das Polypeptid von Anspruch 15, worin die Aminosäuresequenz aus der Gruppe ausgewählt ist, die aus SEQ ID NO: 141 bis SEQ ID NO: 148 besteht.

18. Das Polypeptid von Anspruch 15, worin die Aminosäuresequenz aus der Gruppe ausgewählt ist, die aus SEQ ID NO: 150 bis SEQ ID NO: 239 besteht.

19. Das Polypeptid von Anspruch 15, worin die Aminosäuresequenz aus der Gruppe ausgewählt ist, die aus SEQ ID NO: 5 und SEQ ID NO: 7 bis SEQ ID NO: 18 besteht.

20. Das Polypeptid von Anspruch 15, worin die Aminosäuresequenz SEQ ID NO: 150 ist.

21. Das Polypeptid von Anspruch 15, worin die Aminosäuresequenz SEQ ID NO: 151 ist.

22. Das Polypeptid von Anspruch 15, worin die Aminosäuresequenz SEQ ID NO: 152 ist.

23. Das Polypeptid von Anspruch 15, worin die Aminosäuresequenz SEQ ID NO: 239 ist.

## Revendications

1. Un procédé de production d'une banque de soustraction de substrats de peptides où chaque peptide est un substrat pour une première enzyme modificatrice des peptides mais n'est pas un substrat pour une seconde enzyme modificatrice des peptides, comprenant les étapes consistant à:
a) prévoir une banque combinatoriale comprenant des composés qui affichent différents peptides;
b) mettre en contact la banque combinatoire avec une première enzyme modificatrice des peptides pour permettre à la première enzyme de modifier les peptides de la banque afin de former une première partie de composés de peptides modifiée et une première partie de composés de peptides non modifiée de la banque;
c) séparer la première partie modifiée de la banque et la première partie non modifiée de la banque;
d) mettre en contact la première partie modifiée de la banque avec une seconde enzyme modificatrice des peptides pour permettre à la seconde enzyme de modifier les peptides de la première partie modifiée de la banque afin de former une seconde partie de composés de peptides modifiée et une seconde partie de composés de peptides non modifiée de la banque;
e) séparer la seconde partie modifiée de la banque et la seconde partie non modifiée de la banque; et
f) retenir la seconde partie non modifiée pour former une banque de peptides qui ont chacun un substrat pour la première enzyme mais non pas un substrat pour la seconde enzyme.

2. Le procédé de la revendication 1, dans lequel la première enzyme et la seconde enzyme sont des protéases.

3. Le procédé de la revendication 1, dans lequel la première enzyme comprend un activateur du plasminogène des tissus (t-PA) et la seconde enzyme comprend un activateur du plastinogène de type urokinase (u-PA).

4. Le procédé de la revendication 1, dans lequel la première enzyme comprend un activateur du plasminogène de type urokinase (u-PA) et la seconde enzyme comprend un activateur du plasminogène des tissus (t-PA).

5. Le procédé de la revendication 1, dans lequel la première enzyme et la seconde enzyme sont des kinases.

6. Le procédé de la revendication 1, dans lequel la première enzyme et la seconde enzyme sont des phosphatases.

7. Le procédé de la revendication 1, dans lequel la banque combinatoire est une banque d'affichage de bactériophages.

8. Un procédé de production d'une banque de soustraction de substrats de peptides où chaque peptide est un substrat pour une seconde enzyme modificatrice des peptides mais n'est pas un substrat pour une première enzyme modificatrice des peptides, comprenant les étapes consistant à:
a) prévoir une banque combinatoire comprenant des composés qui affichent différents peptides;
b) mettre en contact la banque combinatoire avec une première enzyme modificatrice des peptides pour permettre à la première enzyme de modifier les peptides de la banque afin de former une première partie de composés de peptides modifiée et une première partie de composés de peptides non modifiée de la banque;
c) séparer la première partie modifiée de la banque et la première partie non modifiée de la banque;
d) mettre en contact la première partie non modifiée de la banque avec une seconde enzyme modificatrice des peptides pour permettre à la seconde enzyme de modifier les peptides afin de former une seconde partie de composés de peptides modifiée et une seconde partie de composés de peptides non modifiée de la banque; et
e) séparer la seconde partie modifiée de la banque et la seconde partie non modifiée de la banque, et retenir la seconde partie modifiée pour former une banque de peptides qui ont chacun un substrat pour la seconde enzyme mais non pas un substrat pour la première enzyme.

9. Le procédé de la revendication 8, dans lequel la première enzyme et la seconde enzyme sont des protéases.

10. Le procédé de la revendication 8, dans lequel la première enzyme comprend un activateur du plasminogène des tissus (t-PA) et la seconde enzyme comprend un activateur du plasminogène de type urokinase (u-PA).

11. Le procédé de la revendication 8, dans lequel la première enzyme comprend un activateur du plasminogène de type urokinase (u-PA) et la seconde enzyme comprend un activateur du plasminogène des tissus (t-PA).

12. Le procédé de la revendication 8, dans lequel la première enzyme et la seconde enzyme sont des kinases.

13. Le procédé de la revendication 8, dans lequel la première enzyme et la seconde enzyme sont des phosphatases.

14. Le procédé de la revendication 8, dans lequel la banque combinatoire est une banque d'affichage de bactériophages.

15. Un polypeptide isolé, dont la séquence d'acides aminés est choisie dans le groupe se composant de SEQ ID NO:2 à SEQ ID NO:5, SEQ ID NO:7 à SEQ ID NO:148 et SEQ ID NO:150 à SEQ ID NO:239.

16. Le polypeptide de la revendication 15, dans lequel la séquence d'acides aminés est choisie dans le groupe se composant de SEQ ID NO:2 à SEQ ID NO:5 et SEQ ID NO:7 à SEQ ID NO:30.

17. Le polypeptide de la revendication 15, dans lequel la séquence d'acides aminés est choisie dans le groupe se composant de SEQ ID NO:141 à SEQ ID NO:148.

18. Le polypeptide de la revendication 15, dans lequel la séquence d'acides aminés est choisie dans le groupe se composant de SEQ ID NO:150 à SEQ ID NO:239.

19. Le polypeptide de la revendication 15, dans lequel la séquence d'acides aminés est choisie dans le groupe se composant de SEQ ID NO:5 et SEQ ID NO:7 à SEQ ID NO:18.

20. Le polypeptide de la revendication 15, dans lequel la séquence d'acides aminés est SEQ ID NO:150.

21. Le polypeptide de la revendication 15, dans lequel la séquence d'acides aminés est SEQ ID NO:151.

22. Le polypeptide de la revendication 15, dans lequel la séquence d'acides aminés est SEQ ID NO:152.

23. Le polypeptide de la revendication 15, dans lequel la séquence d'acides aminés est SEQ ID NO:239.
